# EUROPEAN PATENT SPECIFICATION

(11) **EP 2 528 452 B1**
(45) Date of publication and mention of the grant of the patent: **22.04.2015**
(21) Application number: 11707238.9
(22) Date of filing: 28.01.2011
(51) Int. Cl.: A23K 1/06, B01D 11/02, C12P 7/06, C12P 7/10, C12M 1/00

(54) **APPARATUS AND METHOD FOR TREATING WET SOLIDS FROM A FERMENTATION PROCESS**
VORRICHTUNG UND VERFAHREN ZUR BEARBEITUNG FEUCHTER FESTSTOFFE AUS EINEM FERMENTIERUNGSVERFAHREN
APPAREIL ET PROCÉDÉ POUR TRAITER DES SOLIDES HUMIDES PROVENANT D'UN PROCESSUS DE FERMENTATION

(30) Priority: 29.01.2010 US 299893 P
(43) Date of publication of application: 05.12.2012
(73) Proprietor: POET Research, Inc., Sioux Falls, South Dakota 57104 (US)
(72) Inventor: REDFORD, Steven G., Brandon, South Dakota 57005 (US)
(74) Representative: Gibson, Mark
(86) International application number: PCT/US2011/023025
(87) International publication number: WO 2011/094614

(56) References cited:
- WO-A2-2010/075541
- JP-A- 60 106 820
- US-A- 4 857 279
- US-A- 5 620 728

## Description

### BACKGROUND

Ethanol can be produced from grain-based feedstocks (such as corn), cellulosic feedstocks (such as switchgrass or corn cobs), or other plant material (such as sugar cane).

In a conventional ethanol plant producing ethanol from corn, corn kernels are processed to separate the starch-containing material (e.g. endosperm) from other matter (such as fiber and germ). The starch-containing material is then slurried with water and liquefied to facilitate saccharification where the starch is converted into sugar (i.e. glucose) and fermentation where the sugar is converted by an ethanologen (i.e. yeast) into ethanol. The product of fermentation is beer, which comprises a liquid component containing ethanol and water (among other things) and a solids component containing unfermented particulate matter (among other things).

According to the process typically used at a conventional ethanol plant, the liquefaction of the starch-containing material is done by "cooking" the slurry at temperature at or near the boiling point of water (e.g., in a range of 60-80 °C or greater). According to an alternative process (that has been developed and implemented by the assignee of the present application), for example, as described in U.S. Patent Application Publication No. 2005/0239181, raw starch may be converted and fermented without "cooking" or liquefication.

In a conventional ethanol plant, the liquid component and solids component of the fermentation product is sent to a distillation system. In distillation, the fermentation product is processed into, among other things, ethanol and stillage containing wet solids (i.e. the solids component of the beer with substantially all ethanol removed) formed into a wet cake which can be dried into distillers dried grains (DDG) and sold as an animal feed product. Other co-products, for example, syrup (and oil contained in the syrup) can also be recovered from the stillage. Water removed from the fermentation product in distillation can be treated for re-use at the plant. Treatment of wet solids using a belt conveyor has been described in US-A-5620728.

In a conventional ethanol plant, certain plant operations are conducted at elevated temperatures over ambient temperature with the resultant consumption of energy. For example, the liquefaction of the starch-containing slurry is typically done with a jet cooker (using natural gas as a fuel to elevate the temperature of the slurry to a boil). The amount of energy used in the distillation process (another operation performed at an elevated temperature, with heat typically provided by steam from an on-site boiler) is a function, among other things, of the volume/mass of material supplied to the distillation system. Additionally, the drying of wet solids into distillers dried grains, an operation in which water is removed from the solids typically in a natural gas fired dryer, also consumes energy as a function of the properties (e.g. heat capacity, heat of vaporization and boiling point) of the water to be removed from the solids.

In view of the above, it would be advantageous provide for a system for producing ethanol that facilitates an overall reduction in the use of energy at the plant, for example, by reducing the mass of wet solids supplied to the distillation system. It would also be advantageous to provide for a system for producing ethanol that reduced the amount of energy used to dry the wet solids component of the fermentation product. It would further be advantageous to provide for a system for producing ethanol that facilitated the recovery of co-products including bioproducts and other biochemicals extracted from components of the fermentation product. It would further be advantageous to provide for a system for producing ethanol in which the solids component of the fermentation product would be dried and constituted into a meal that could be used for animal feed, among other uses.

### SUMMARY

In one embodiment, the invention provides an apparatus for treating a layer of wet solids (e.g., from a fermentation process) with one or more liquids, the apparatus comprising:
(a) a belt having a first major surface and a second major surface and a plurality of pores extending between the first major surface and the second major surface to provide the belt with porosity to the one or more liquids;
(b) a drive system for advancing the belt in indexing movements in a downweb direction;
(c) a treatment chamber configured for movement between: (i) a first position where the treatment chamber is separated from the belt in order to allow the belt to be advanced by the drive system in indexing movements in a downweb direction; and (ii) a second position where the treatment chamber is in sealing contact with the first major surface of the belt or with the layer of particulate solids positioned on the first major surface of the belt;
(d) one or more nozzles in the treatment chamber for applying one or more liquids to the wet solids; and
(e) one or more reservoirs for holding one or more liquids for treatment of the wet solids; wherein the one or more reservoirs are in fluid communication with the one or more nozzles.

Optionally, the apparatus may further include a vacuum apparatus positioned proximate the second major surface of the belt for removing at least a portion of liquid applied to the wet solids through the pores in the belt to form an effluent.

In another embodiment, the invention provides a method of treating a wet solid with one or more liquids to form a treated solid, the method comprising the steps of:
(a) providing an apparatus as described herein;
(b) applying wet solids to the first major surface of the belt to form a layer of wet solids;
(c) moving the treatment chamber from (i) a first position where the treatment chamber is separated from the belt to (ii) a second position where the treatment chamber is in sealing contact with the first major surface of the belt or with the layer of wet solids;
(d) applying one or more liquids to the layer of wet solids positioned under the treatment chamber while the treatment chamber is in sealing contact; and
(e) at least partially removing the one or more liquids from the wet solids (e.g., by applying vacuum to the second major surface of the belt) to form an effluent.

In yet another embodiment, the invention provides a method of treating wet solids with one or more liquids to form a treated solid, the method comprising the steps of:
(a) providing an apparatus as described herein; wherein the apparatus comprises a first treatment chamber and a second treatment chamber;
(b) applying wet solids to the first major surface of the belt to form a layer of wet solids;
(c) moving the first treatment chamber and the second treatment chamber into sealing contact with the first major surface of the belt or with the layer of wet solids;
(d) applying a first liquid to the layer of wet solids under the first treatment chamber;
(e) sequentially applying two or more liquids to the layer of wet solids under the second treatment chamber;
(f) at least partially removing at least a portion of the liquids applied in steps (d) and (e) from the wet solids;
(g) moving the first and second treatment chambers out of sealing contact with the belt; and
(h) advancing the belt a distance in the downweb direction.

In an exemplary embodiment, steps (d), (e), and (f) are performed during approximately the same time period so that, upon completion of step (f), the wet solids under the first treatment chamber have been treated by the first liquid, and the wet solids under the second treatment chamber have been treated by the two or more liquids.

### BRIEF DESCRIPTION OF THE FIGURES

FIGURE 1 is a process flow diagram of an exemplary embodiment of a biorefinery.
FIGURE 2 is a process flow diagram of an exemplary embodiment of the biorefinery of FIGURE 1.
FIGURE 3 is a process flow diagram of an exemplary embodiment of the biorefinery of FIGURE 1.
FIGURE 4 is a process flow diagram of an exemplary embodiment of the biorefinery of FIGURE 1.
FIGURE 5 is a process flow diagram of an exemplary embodiment of the biorefinery of FIGURE 1.
FIGURE 6 is a block flow diagram of an exemplary embodiment of the biorefinery.
FIGURE 7 is a block flow diagram of an exemplary embodiment of the biorefinery.
FIGURE 8 is a block flow diagram of an exemplary embodiment of the biorefinery.
FIGURE 9 is a block flow diagram of an exemplary embodiment of the biorefinery.
FIGURE 10 is a block flow diagram of an exemplary embodiment of the biorefinery.
FIGURE 11 is a block flow diagram of an exemplary embodiment of the biorefinery.
FIGURE 12 is a block flow diagram of an exemplary embodiment of the biorefinery.
FIGURE 13 is a block flow diagram of an exemplary embodiment of solids washing processes.
FIGURE 14 is a process flow diagram of an exemplary embodiment of the solids washing processes.
FIGURE 15 is an exemplary graphical representation of energy for drying wet solids vs. the ethanol concentration of the wet solids.
FIGURE 16 is a block flow diagram of an exemplary embodiment of the solids washing processes.
FIGURE 17 is a process flow diagram of an exemplary embodiment of the solids washing processes.
FIGURES 18A and 18B are cross-sectional views of an exemplary embodiment of a wash process capable of implementing multiple wash stages.
FIGURES 19 through 23 are cross-sectional views at various locations along the filter belt of FIGURES 18A and 18B.
FIGURE 24 is a perspective view of an exemplary embodiment of the ethanol wash process.
FIGURES 25A through 25E are schematic cross-sectional views of an exemplary embodiment of a wash apparatus and process capable of implementing multiple stage, single chamber washing.
FIGURE 25F is a schematic perspective view of an embodiment of a filter belt.
FIGURE 26 is a schematic cross-sectional view of an exemplary embodiment of an apparatus and wash process of FIGURES 25A through 25E during the exemplary ethanol washing sequence (i.e., FIGURE 25C).
FIGURES 27A through 27F are schematic cross-sectional views of an exemplary embodiment of a single chamber wash process.
FIGURE 28 is a schematic cross-sectional view of an exemplary embodiment of an apparatus and process of the invention.
FIGURE 29 is a schematic cross-sectional view of an exemplary embodiment of an apparatus and process of the invention.

### DETAILED DESCRIPTION

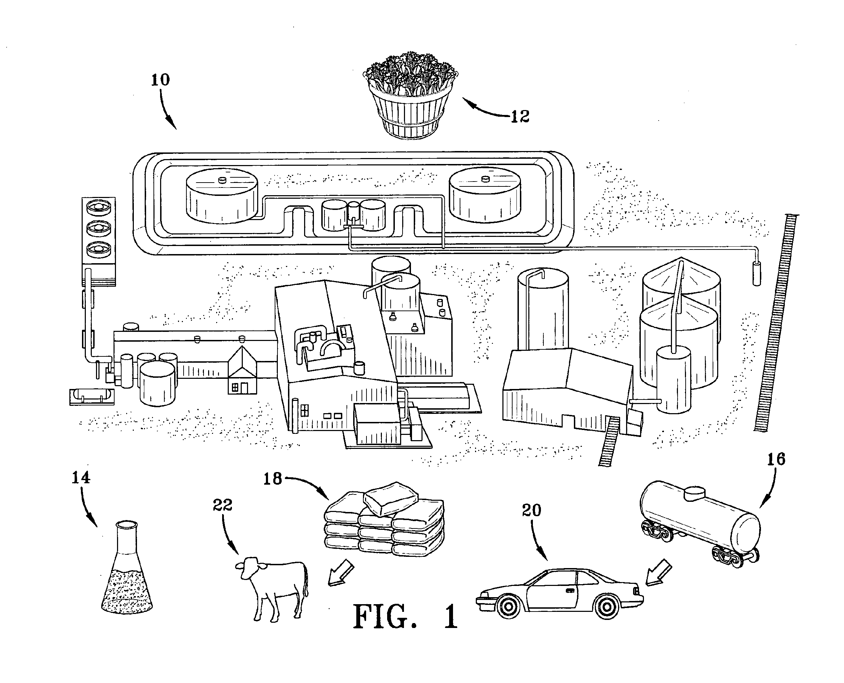

FIGURE 1 is a process flow diagram of an exemplary embodiment of a biorefinery 10. Biorefinery 10 receives a feedstock, illustrated as corn 12, and processes the feedstock to produce several usable products for consumption, principally ethanol. Although illustrated as corn 12, other types of feedstock such as sorghum, wheat, barley, potatoes, sugar cane, switchgrass, and corn cobs, may be processed by biorefinery 10. Additional inputs such as enzymes, yeast, water, and energy (e.g., heat energy) may be added to the feedstock to facilitate production of the usable products. The usable products from biorefinery 10 may include bioproducts 14, such as corn oil, corn syrup, bran, flour, proteins (e.g., zein), and other suitable bioproducts, ethanol 16, and an animal feed 18 shown as distillers dried grain (DDG).

Ethanol 16 is an alcohol produced from corn 12 or other starch-based crop. Ethanol 16 has many uses, and of particular interest is its capacity to be blended with gasoline for use in motor vehicles 20. Ethanol 16 is a relatively clean-burning, high-octane fuel that may be produced domestically in the United States from renewable sources, reducing the dependence on foreign sources of energy. Ethanol 16 also delivers economic vitality to agricultural regions where the feedstocks are produced. Ethanol blends increase fuel octane ratings, decrease harmful fossil fuel emissions, reduce fuel costs, and extend the overall supply of gasoline.

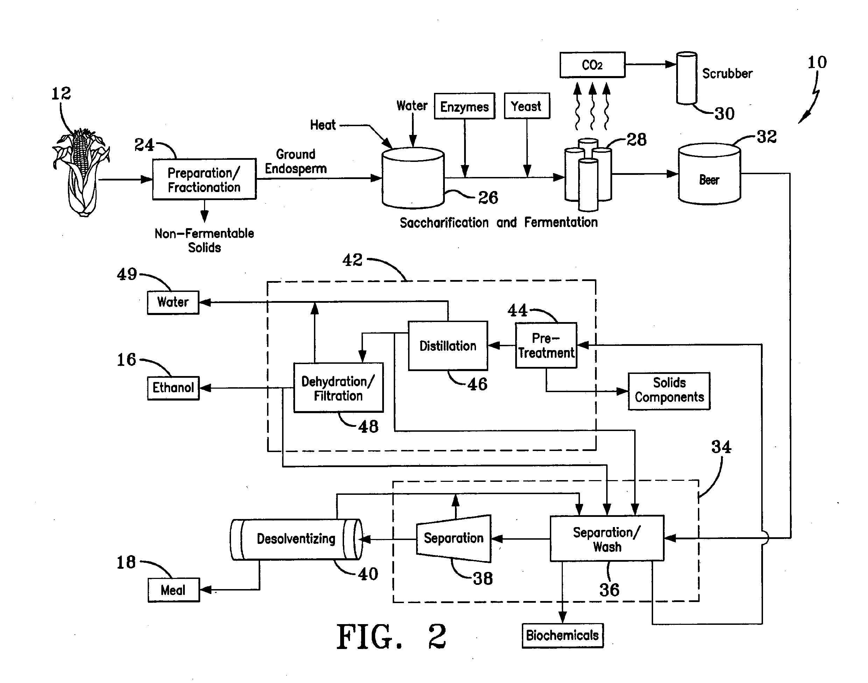

FIGURE 2 is a process flow diagram of an exemplary embodiment of biorefinery 10 of FIGURE 1. Corn 12 may be directed into a preparation/fractionation process 24, where corn kernels of corn 12 may be separated into non-fermentable solids (e.g., germ and fiber) and fermentable solids (e.g., endosperm). Once the endosperm has been separated from the non-fermentable solids, the endosperm may be ground into ground endosperm, which may be directed into a saccharification process 26. Separation and grinding of endosperm may also be conducted in an integrated process. Saccharification process 26 may also receive additional inputs (e.g., heat, water, and enzymes) and may convert starches within the ground endosperm into sugars, which may be suitable for fermenting. A fermentation slurry may be directed from saccharification process 26 into a fermentation process 28.

Fermentation process 28 may also receive additional inputs (e.g., yeast and enzymes) and may ferment the sugars within the fermentation slurry to produce a certain concentration of ethanol within the fermentation slurry. Fermentation process 28 may produce a certain amount of carbon dioxide (CO₂) and other gases, which may be processed through the use of a scrubber 30 or other suitable equipment. The main product of fermentation process 28 is a fermentation product shown as beer 32 comprises a liquid component and a solids component and is generally a mixture of ethanol, water, syrup, particulate matter, and dissolved solids. Saccharification process 26 and fermentation process 28 may be performed separately, or according to certain embodiments, may be combined into a substantially integrated process (e.g., called simultaneous saccharification and fermentation (SSF)).

Beer 32 may be directed into solids processing system 34, which may wash beer 32 with ethanol (or other solvent). Solids processing system 34 of FIGURE 2 include a separation/wash process 36, a separation process 38, and a desolventizing process 40. Beer 32 may be separated into a liquid component and a solids component by separation/wash process 36. The liquid component from separation/wash process 36 may be processed by a series of distillation system 42, which primarily produce ethanol 16. Distillation system 42 may include a distillation pre-treatment process 44, a distillation process 46, and a dehydration/filtration process 48. Distillation pre-treatment process 44 may remove wet solids components from the liquid component before distillation process 46 produces ethanol, which may be dried within dehydration/filtration process 48 to remove any remaining water 49. Dehydration/filtration process 48 may include any suitable type of dehydration, such as dessication. Water 49 removed from distillation system 42 may be used as make-up water, as a slurry water source, or as a source of water for other processes internal or external to biorefinery 10.

The solids component comprises ethanol, water, syrup, meal, zein, lutein, lysine, various proteins (having different attributes and nutritional values), yeast, fiber, and other particulate matter and dissolved solids. The solids component may be processed through solids processing system 34, which primarily produces meal 18 and several biochemicals, such as zein and xanthophylls. Ethanol 16 from distillation process 46 and/or dehydration/filtration process 48 may be used in separation/wash process 36 to wash the solids component with ethanol, increasing the ethanol concentration of the solids component and reducing the energy required to desolventize the solids component in the desolventizing process 40 to produce meal 18. Liquids removed by separation process 38 and desolventizing process 40 may be directed to separation/wash process 36.

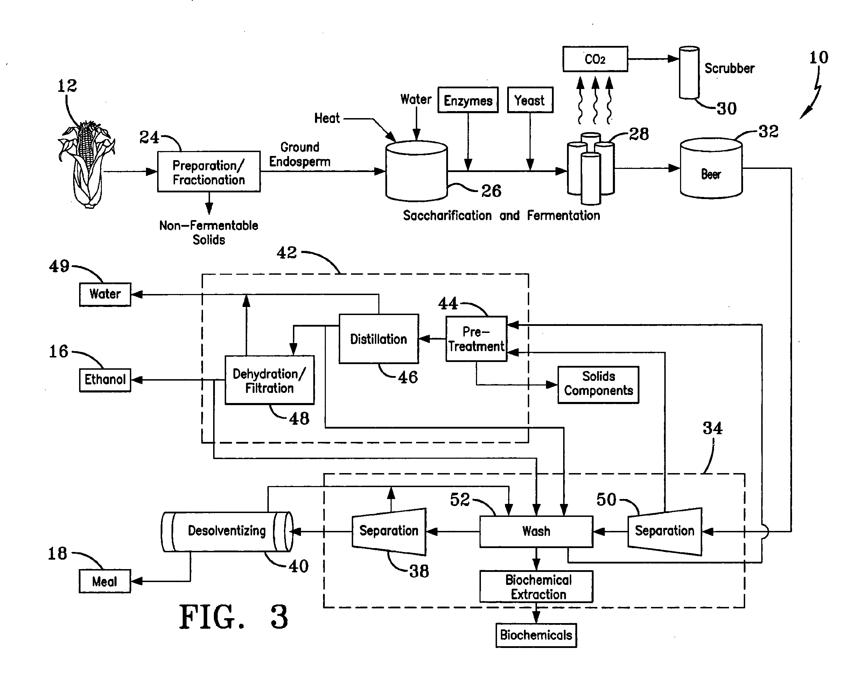

FIGURE 3 is a process flow diagram of an exemplary embodiment of biorefinery 10 of FIGURE 1. The processes are substantially similar to those of FIGURE 2 through the production of beer 32. Beer 32 may be directed into a separation process 50, which separates the beer 32 into a liquid component and a solids component. The liquid component from separation process 50 may be processed by distillation processes operating through distillation system 42 to produce ethanol 16.

The solids component may be directed into a wash process 52, which washes the solids component with ethanol 16 from distillation process 46 and/or dehydration/filtration process 48. Biochemicals removed by wash process 52 may be extracted by a biochemical extraction process. Certain components from wash process 52 may be directed into distillation system 42 (e.g., distillation pre-treatment process 44). Ethanol-washed solids from wash process 52 may be directed into separation process 38, where a certain amount of water and ethanol may be removed before the ethanol (e.g., solvent) is removed by desolventizing process 40 to produce meal 18. Liquids removed by separation process 38 and desolventizing process 40 may be directed to wash process 52.

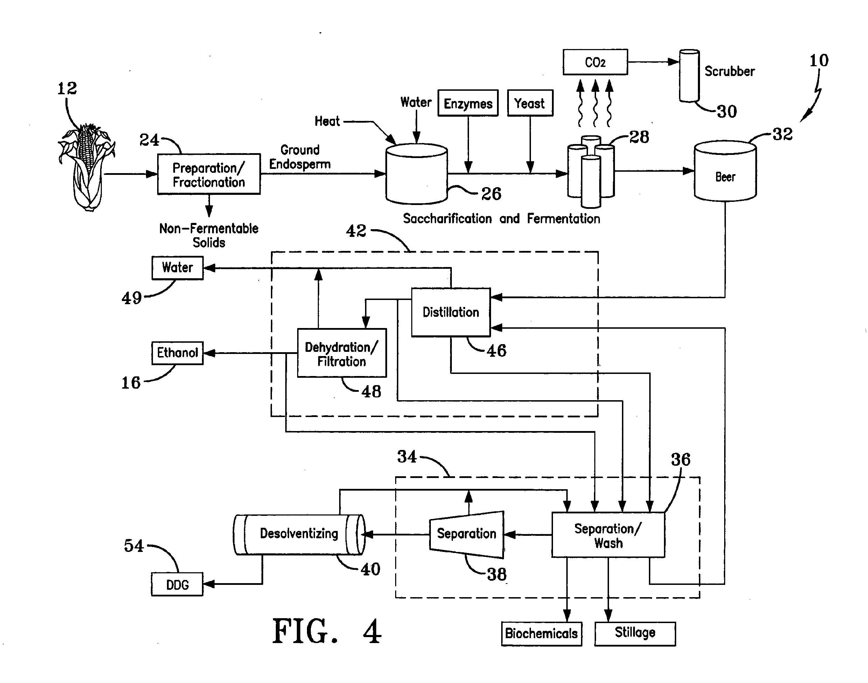

FIGURE 4 is a process flow diagram of an exemplary embodiment of biorefinery 10 of FIGURE 1. The processes are substantially similar to those of FIGURES 2 and 3 through the production of beer 32. Beer 32 may be directed into distillation process 46, with ethanol from distillation process 46 being directed to dehydration/filtration process 48, and the liquid/solids mixture (e.g., stillage) from distillation process 46 being directed into separation/wash process 36. Separation/wash process 36 may receive ethanol from distillation process 46 and/or dehydration/filtration process 48 and wash the liquid/solids mixture (e.g., stillage) from distillation process 46 with the ethanol, increasing the ethanol concentration of the liquid/solids mixture (e.g., stillage). The ethanol-washed liquid/solids mixture (e.g., stillage) may be directed into separation process 38, where a certain amount of water and ethanol may be removed before the ethanol (e.g., solvent) is removed by desolventizing process 40 to produce distillers dried grain (DDG) 54. Liquids removed by separation process 38 and desolventizing process 40 may be recycled back through separation/wash process 36. Biochemicals and stillage may be extracted from separation/wash process 36.

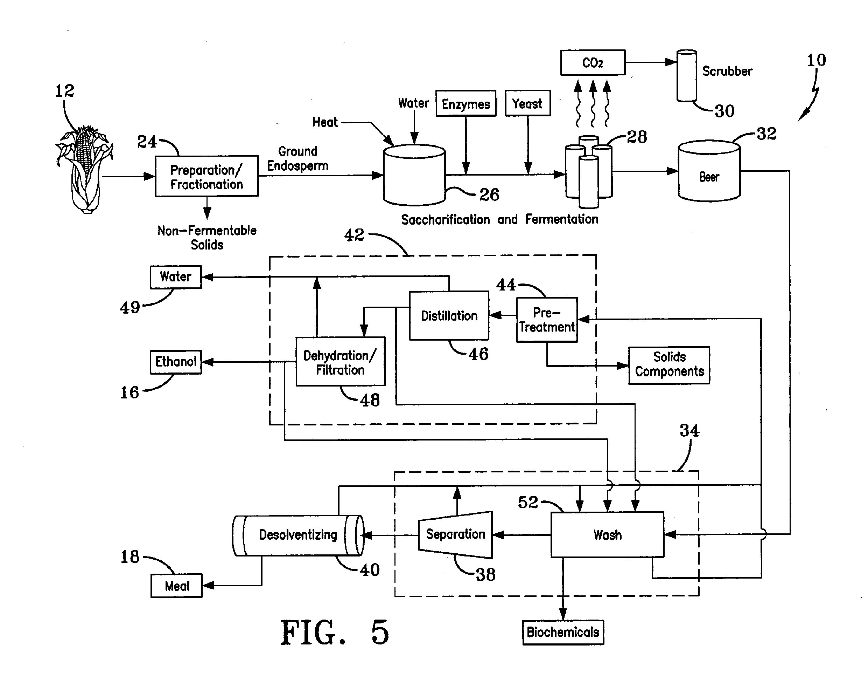

FIGURE 5 is a process flow diagram of an exemplary embodiment of biorefinery 10 of FIGURE 1. The processes are substantially similar to those of FIGURES 2 through 4 through the production of beer 32. Beer 32 may be directed into wash process 52, which may wash beer 32 with ethanol from distillation process 46 and/or dehydration/filtration process 48. The ethanol-washed beer 32 may be directed into separation process 38, where a certain amount of water and ethanol may be removed before the ethanol (e.g., solvent) is removed by desolventizing process 40 to produce meal 18. Liquid removed by separation process 38 and desolventizing process 40 may be recycled back through wash process 52. Ethanol and water from wash process 52, separation process 38, and desolventizing process 40 may also be directed into distillation system 42 (e.g., distillation pre-treatment process 44).

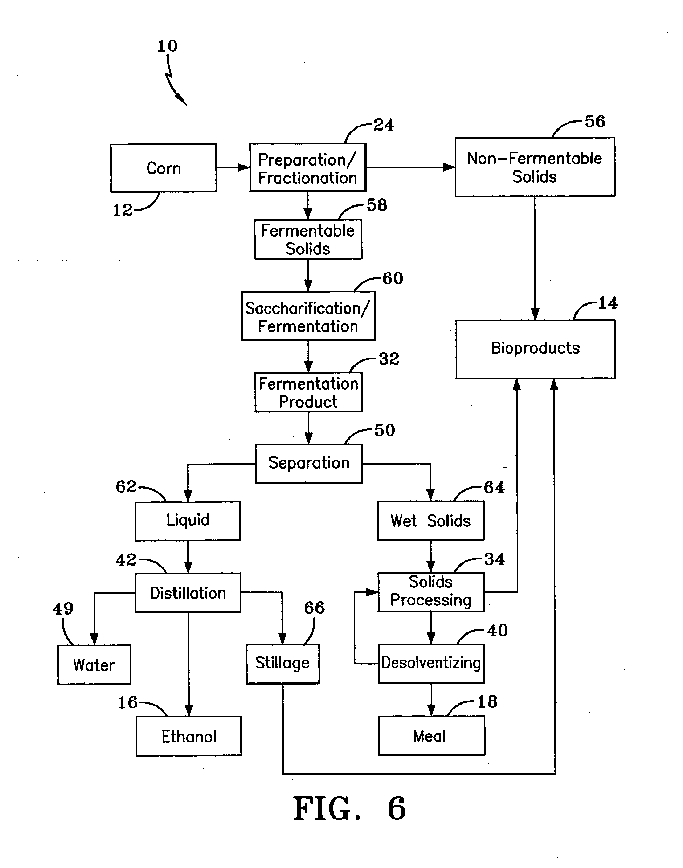

FIGURE 6 is a block flow diagram of an exemplary embodiment of biorefinery 10. Corn 12 may first be directed into preparation/fractionation process 24, where it is prepared for saccharification and fermentation. Non-fermentable solids in corn 12 may be separated (e.g., fractionated) from fermentable solids. Corn kernels generally comprise endosperm, germ, and fiber. Endosperm comprises most of the starches and proteins available in a corn kernel and, therefore, is used in fermentation process 28 to generate ethanol. In other words, endosperm represents the fermentable solids of a corn kernel; germ and fiber represent the non-fermentable solids, which may be withheld from fermentation process 28. Endosperm comprises approximately 80-85% of a corn kernel, germ comprises approximately 10-15% of a corn kernel, and fiber comprises approximately 5-10% of a corn kernel, all by mass.

Non-fermentable solids 56 may be directed into various bioproduct processes, which may produce usable bioproducts 14. Non-fermentable solids 56 may include the germ and fiber of corn 12, which may be processed into bioproducts such as corn oil, corn syrup, bran, flour, and proteins (e.g., zein). Fermentable solids 58 (e.g., endosperm of corn 12) from preparation/fractionation process 24 may be directed into a saccharification/fermentation process 60, which may include saccharification process 26 and fermentation process 28. Saccharification process 26 and fermentation process 28 may be conducted separately (e.g., in separate stages) or may be conducted concurrently (e.g., in an integrated stage). It should be noted that fermentable solids 58 may contain small portions of non-fermentable components (e.g., germ and fiber) not intended for the fermentation process.

Preparation/fractionation process 24 may include passing corn 12 through mills, such as hammer mills and pins mills, to grind fermentable solids 58 into a fine powder (e.g., flour), further facilitating saccharification/fermentation process 60. Fermentable solids 58 (e.g., endosperm) include a high proportion of starches suitable for fermenting to produce ethanol 16. Saccharification/fermentation process 60 may include saccharifying fermentable solids 58 to convert the starches within fermentable solids 58 into sugars. The process of saccharifying fermentable solids 58 may include adding heat, water, and enzymes to fermentable solids 58 to produce a fermentation slurry.

Saccharification/fermentation process 60 may also include adding yeast to the fermentation slurry. The yeast helps convert the sugars within the fermentation slurry into ethanol 16 and carbon dioxide. The fermentation slurry may be agitated and cooled until the concentration of ethanol 16 has been maximized. The output from saccharification/fermentation process 60 may be referred to as fermentation product 32, which may generally include ethanol 16, but may also include a certain amount of water, as well as syrup, particulate matter, and dissolved solids.

Fermentation product may be separated by separation process 50 into a liquid component and a solids component, both of which may be processed in respective processing paths. The liquid component may include liquid 62, which contains ethanol 16, a certain amount of water and other non-ethanol liquids, as well as fine solids, which may be removed from liquid 62. Distillation system 42 may remove most of the water, other non-ethanol liquids, and fine solids to produce ethanol 16. Ethanol 16 leaving distillation system 42 may contain various target concentrations of ethanol, such as from approximately 95% (e.g., 190 proof) to approximately 100% (e.g., 200 proof). Stillage 66 (e.g., comprising liquid and wet solids) from distillation system 42 may be processed and/or combined into bioproducts 14 (e.g., animal feed, oils, syrup, and other biochemicals).

The solids component may include wet solids 64, which may include a certain amount of ethanol, a certain amount of water, syrup, particulate matter, and dissolved solids. It should be noted that when reference is made to "solids," the solids may include particulate matter and dissolved solids, which may be associated with a certain amount of liquids (e.g., "wet solids"). Solids processing system 34 and desolventizing process 40 may remove most of the water and ethanol from wet solids 64 to produce meal 18. Solids processing system 34 may include washing wet solids 64 with ethanol or a liquid with a desired ethanol content to decrease the boiling point, specific heat, and enthalpy (heat) of vaporization of the wet solids 64, reducing the energy required to dry (e.g., desolventize) wet solids 64 to produce meal 18. The ethanol may be directed to solids processing system 34.

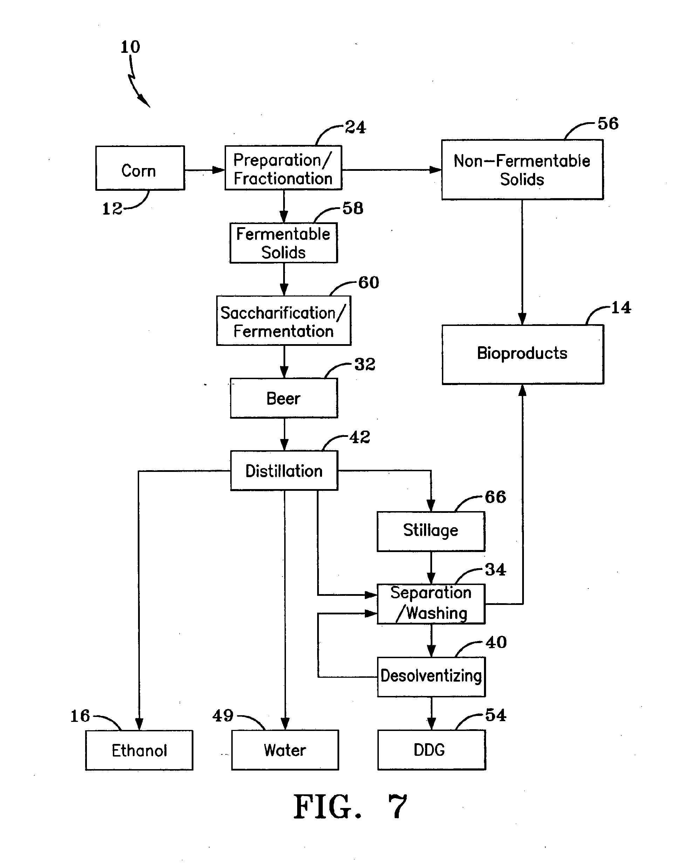

FIGURE 7 is a block flow diagram of an exemplary embodiment of biorefinery 10. The processes are substantially similar to those of FIGURE 6 through the production of beer 32. The fermentation product shown as beer 32 may be directed into distillation system 42, where beer 32 is distilled to produce ethanol 16. Stillage 66 from distillation system 42 may be directed into solids processing system 34 (e.g., including separation and washing), where stillage 66 may be washed with ethanol from distillation system 42 and/or desolventizing process 40 to increase the ethanol concentration of stillage 66, reducing the amount of energy required by desolventizing process 40 to remove liquids from stillage 66 to produce DDG 54. Thin stillage may be removed from solids processing system 34 as bioproducts 14.

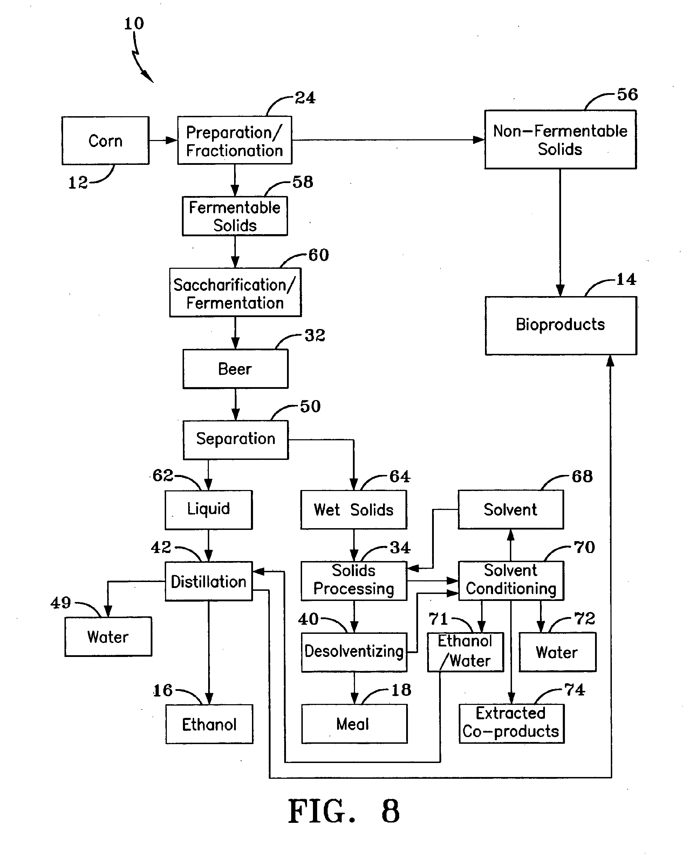

FIGURE 8 is a block flow diagram of an exemplary embodiment of biorefinery 10. The processes are substantially similar to those of FIGURES 6 and 7 through the production of the fermentation product shown as beer 32. Beer 32 may be separated by separation process 50 into a liquid component shown as comprising liquid 62 and a solids component shown as comprising wet solids 64. Ethanol 16 may be recovered from liquid 62 by distillation system 42; wet solids 64 may be directed into solids processing system 34, where solids may be washed with a solvent 68 (e.g., hexane) other than ethanol. Solvent 68 may be received by solids processing system 34 from a solvent conditioning process 70, which may in turn receive spent solvent from solids processing system 34, forming a closed-loop cycle of solvent 68 through solids processing system 34. Solvent from solvent-washed wet solids 64 may be removed by desolventizing process 40 to produce meal 18. Solvent conditioning process 70 may also remove a ethanol/water mixture 71 and direct the ethanol/water mixture 71 to distillation system 42. Solvent conditioning process 70 may further remove water 72 and extracted co-products 74 from wet solids 64.

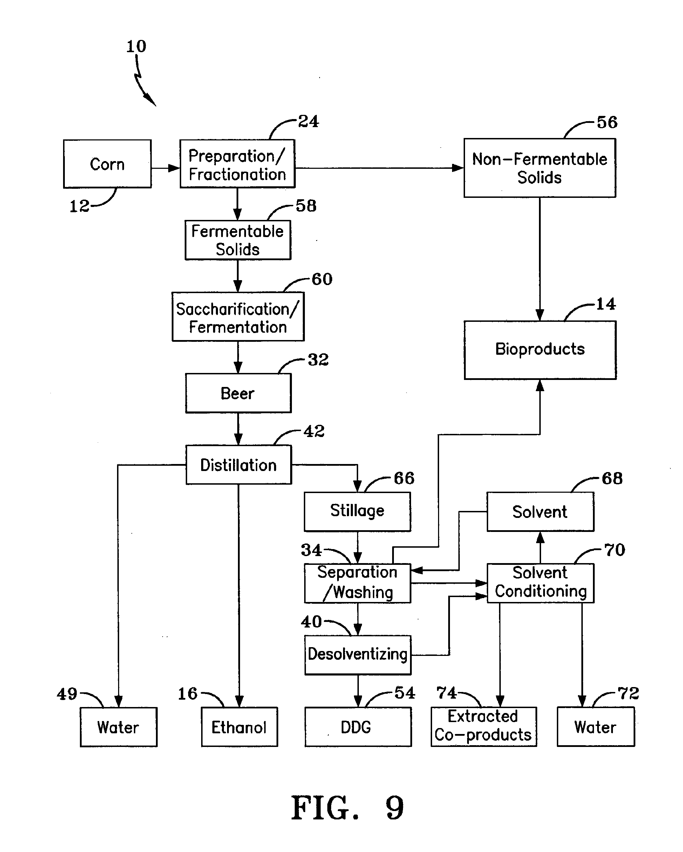

FIGURE 9 is a block flow diagram of an exemplary embodiment of biorefinery 10. The processes are substantially similar to those of FIGURES 6 through 8 through the production of the fermentation product shown as beer 32. Beer 32 may be directed into distillation system 42, where beer 32 is distilled to produce ethanol 16. Stillage 66 from distillation system 42 may be directed into solids processing system 34 (e.g., separation and washing), where stillage 66 may be washed with solvent 68 (e.g., hexane). Solvent 68 may be received by solids processing system 34 from solvent conditioning process 70, which may in turn receive spent solvent from solids processing system 34, forming a closed-loop cycle of solvent 68 through solids processing system 34. Solvent from solvent-washed wet solids 64 may be removed by desolventizing process 40 to produce DDG 54. Solvent conditioning process 70 may further remove water 72 and extracted co-products 74 from stillage 66. Thin stillage may be removed from solids processing system 34 as bioproducts 14.

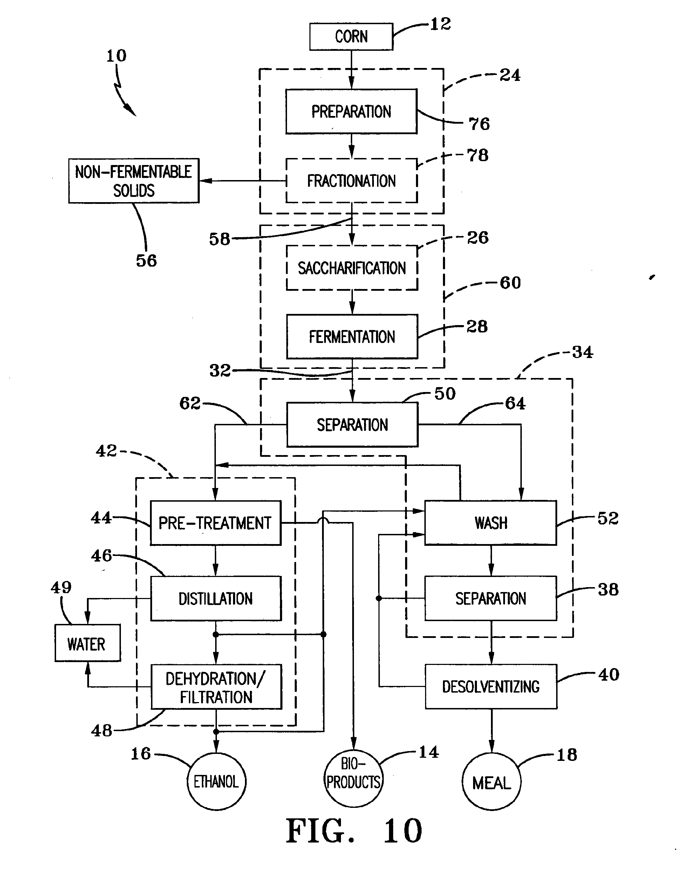

FIGURE 10 is a block flow diagram of an exemplary embodiment of biorefinery 10. Biorefinery 10 begins with preparation/fractionation process 24, which may include a preparation process 76 and/or a fractionation process 78. Preparation/fractionation process 24 prepares corn 12 for saccharification and fermentation in saccharification process 26 and fermentation process 28.

Preparation process 76 may include a cleaning stage to remove impurities that may be present in the corn, such as stalks, cobs, stone, sand, and other fine particles. Clean corn output from the cleaning stage may be directed into a water tempering stage, where corn 12 may be tempered with a water concentration for a period of time. In the water tempering stage, the water penetrates the germ and fiber of corn 12, facilitating subsequent removal of the germ and fiber from corn 12, as well as increasing resistance of the germ and fiber to physical breakage during subsequent stages (e.g., further separation).

Tempered whole corn 12 from preparation process 76 may then be directed into fractionation process 78. Corn 12 may be fractionated into non-fermentable solids 56 (e.g., primarily germ and fiber) and fermentable solids 58 (e.g., primarily endosperm) with fermentable solids 58 being milled to reduce the particle size of fermentable solids 58. The downstream processes of biorefinery 10 may not require that corn 12 be fractionated into endosperm, germ, and fiber. For example, separation process 50 and solids processing system 34 do not require fractionated corn 12 to lead to beneficial results, although such fractionation may enhance the benefits.

The ground endosperm from fractionation process 78 may be directed into saccharification process 26, in which starch within the endosperm may be converted into sugars that can be fermented by a microorganism, such as yeast. The conversion may be accomplished by saccharifying the endosperm with a number of additional inputs, such as saccharifying enzyme compositions, without cooking the endosperm. The downstream processes of biorefinery 10 may not require that corn 12 be saccharified prior to fermentation process 28, although certain benefits and co-products may be enhanced by saccharification process 26.

The output of saccharification process 26 may be described as a fermentation slurry, which may be directed into fermentation process 28, in which sugars within the fermentation slurry are fermented to produce ethanol 16. Additional inputs (e.g., microorganisms such as yeast) may be introduced into fermentation process 28 to facilitate the fermenting. The output of fermentation process 28 may include fermentation product 32, such as a mixture of ethanol, water, syrup, particulate matter (e.g., fiber, germ, yeast, etc.), and dissolved solids.

Fermentation product 32 from fermentation process 28 may be directed into separation process 50, in which fermentation product 32 is separated into a liquid component (e.g., liquid 62) and a solids component (e.g., wet solids 64). The equipment used for separation process 50 may vary and may include, for example, centrifuges, decanters, hydroclones, sedimentation tanks, and filter presses.

Liquid 62 (e.g., water/ethanol mixture) and wet solids 64 (e.g., wet solids) may then be directed into separate processing paths. Liquid 62 may be directed into distillation system 42; wet solids 64 may be processed through solids processing system 34. An end product of the liquids processing path is ethanol 16; an end product of the solids processing path is meal 18 (and possibly other bioproducts such as biochemicals).

Liquid 62 (e.g., water/ethanol mixture) from separation process 50 may first be directed into distillation pre-treatment process 44, in which liquid 62 is prepared for further distillation. Distillation pre-treatment process 44 may include heating liquid 62 prior to distillation. Distillation pre-treatment process 44 may also include removing the remaining fractions of germ and fiber from liquid 62 as bioproducts 14. Once liquid 62 has been pre-treated by distillation pre-treatment process 44, the pre-treated liquid 62 may be directed into distillation process 46, in which water may be removed from ethanol 16. Ethanol 16 from distillation process 46 may be approximately 190 proof (e.g., approximately 95% alcohol). Ethanol 16 from distillation process 46 may then be directed into dehydration/filtration process 48, in which ethanol 16 is further dried and filtered. Ethanol 16 from dehydration/filtration process 48 may be approximately 200 proof (e.g., approximately 100% alcohol). Ethanol 16 from dehydration/filtration process 48 may be sold for use as a fuel.

Wet solids 64 from separation process 50 may first be directed into a wash process 52, in which wet solids 64 are washed with various concentrations of ethanol 16 to increase the ethanol concentration of wet solids 64. The wet solids 64 may then be directed into separation process 38 and desolventizing process 40, in which the wet solids 64 may be deliquified, separated, and desolventized to generate meal 18. By increasing the ethanol concentration of wet solids 64 in wash process 52, the boiling point of the liquid component of the wet solids will be decreased, as will its specific heat and enthalpy (heat) of vaporization, reducing the energy required to dry (e.g., desolventize) the wet solids in desolventizing process 40. Wash process 52 and separation process 38 may be integrated or separate, such that each stage through wash process 52 and separation process 38 progressively increases the ethanol content in the wet solids. Ethanol 16 from separation process 38 and desolventizing process 40 may be used to increase the ethanol concentration of wet solids 64 in wash process 52.

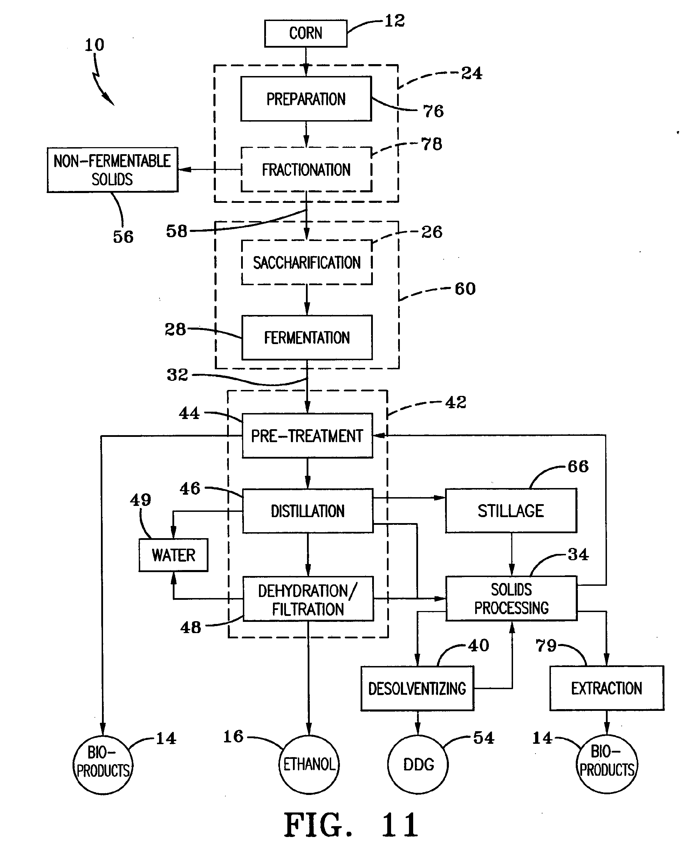

FIGURE 11 is a block flow diagram of an exemplary embodiment of biorefinery 10. The processes are substantially similar to those of FIGURE 10 through fermentation process 28. Fermentation product 32 from fermentation process 28 may be directed into distillation system 42 (e.g., distillation pre-treatment process 44, distillation process 46, and dehydration/filtration process 48), where fermentation product 32 is distilled to produce ethanol 16. Stillage 66 from distillation process 46 may be directed into solids processing system 34, where stillage 66 may be washed with ethanol to increase the ethanol concentration of stillage 66, reducing the amount of energy required by desolventizing process 40 to remove liquids from stillage 66 to produce DDG 54. Solids processing system 34 may receive the ethanol from distillation process 46 and/or dehydration/filtration process 48. A certain amount of ethanol from desolventizing process 40 may be directed back to solids processing system 34 for further use. An extraction process 79 may also extract bioproducts 14 from solids processing system 34. Some biochemicals may be extracted from solids processing system 34 and directed into distillation pre-treatment 44 for further processing.

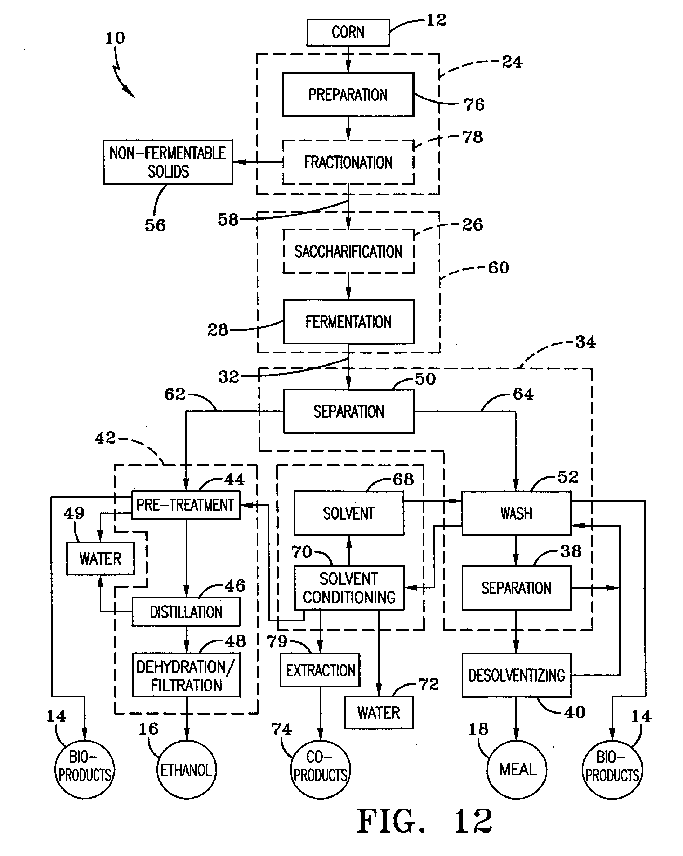

FIGURE 12 is a block flow diagram of an exemplary embodiment of biorefinery 10. The processes are substantially similar to those of FIGURES 10 and 11 through fermentation process 28. Fermentation product 32 from fermentation process 28 may be separated by separation process 50 into a liquid component shown as comprising liquid 62 and a solids component shown as comprising wet solids 64. Liquid 62 may be converted into ethanol 16 by distillation system 42 (e.g., distillation pre-treatment process 44, distillation process 46, and dehydration/filtration process 48); wet solids 64 may be directed into wash process 52, where solids may be washed with a solvent 68 (e.g., hexane) other than ethanol. Solvent 68 may be received by wash process 52 from solvent conditioning process 70, which may in turn receive spent solvent from wash process 52, forming a closed-loop cycle of solvent 68 through wash process 52. Solvent from solvent-washed wet solids 64 may be removed by separation process 38 and desolventizing process 40 to produce meal 18. Some of the solvent removed from separation process 38 and desolventizing process 40 may be directed to wash process 52 for further use. Solvent conditioning process 70 may also remove an ethanol/water mixture 71 and direct the ethanol/water mixture 71 to distillation system 42 (e.g., distillation pre-treatment process 44). Solvent conditioning process 70 may further remove water 72; extraction process 79 may extract co-products 74. Both pre-treatment process 44 and wash process 52 may facilitate the recovery of bioproducts 14 available from the fermentation product 32.

By fractionating and milling the feedstock into primarily ground endosperm in fractionation process 78, the downstream processes of biorefinery 10 may be indirectly enhanced. Because primarily fermentable endosperm is directed into saccharification process 26, only enough energy to saccharify the endosperm will be required by saccharification process 26. Energy need not be expended processing the non-fermentable solids (e.g., germ and fiber) in saccharification process 26 or in other processes, such as the operation of distillation system 42. Because the fermentation slurry from saccharification process 26 consists of primarily fermentable solids, water, and enzymes, fermentation process 28 may also be generally more efficient.

By saccharifying the ground endosperm in saccharification process 26 without cooking, the amount of heat input into the ground endosperm may be lower as compared to conventional cooking processes. Saccharifying the ground endosperm without "cooking" (e.g., using "raw starch" hydrolysis) may lead to meal 18 having a different quality than DDG 54 produced by conventional biorefineries. For example, meal 18 may be higher in protein values, as well as higher in amino acids (e.g., lysine) and other biochemicals having different attributes and qualities, than typical DDG 54.

Separating a liquid component comprising liquid 62 from a solids component comprising wet solids 64 in separation process 50 may lead to several benefits downstream of separation process 50. Because wet solids 64 (e.g., a certain amount of ethanol, a certain amount of water, syrup, particulate matter, and dissolved solids) have been substantially removed from liquid 62 (e.g., a water/ethanol mixture), the distillation equipment of distillation process 46 will be much less likely to encounter fouling from wet solids 64, which may otherwise impair the performance of the distillation equipment, render it less efficient, and/or require cleaning. The distillation equipment of distillation process 46 may also be sized smaller because the added mass of wet solids 64 need not be processed through the distillation equipment. The removal of wet solids 64 may also lead to the distillation equipment of distillation process 46 requiring less energy than conventional distillation equipment.

The combination of the processes of biorefinery 10 may lead to overall energy reduction of biorefinery 10, as well as reducing the temperature to which the products of biorefinery 10 are exposed. Wet solids 64, stillage 66, meal 18, and DDG 54 may all be processed by biorefinery 10 without ever experiencing temperatures above about 150 °C or above about 65° C. Maintaining the temperature below about 150 °C (e.g., below about 100° C or about 65° C) has been shown to reduce the possibility of degradation of the resulting meal 18 or DDG 54. Reduced degradation may include color transformation and significant oxidation of residual starches downstream of fermentation process 28. Using measurements of the resulting meal 18 or DDG 54, such as neutral detergent fiber (NDF) measurements, it has been found that maintaining the temperatures experienced by meal 18 or DDG 54 within biorefinery 10 under approximately 150 °C significantly reduces the possibility of degrading the resultant meal 18 or DDG 54. Using these same measurements, it has been found that maintaining the temperatures experienced by meal 18 or DDG 54 within biorefinery 10 under approximately 100 °C may further reduce the possibility of degrading the meal 18 or DDG 54.

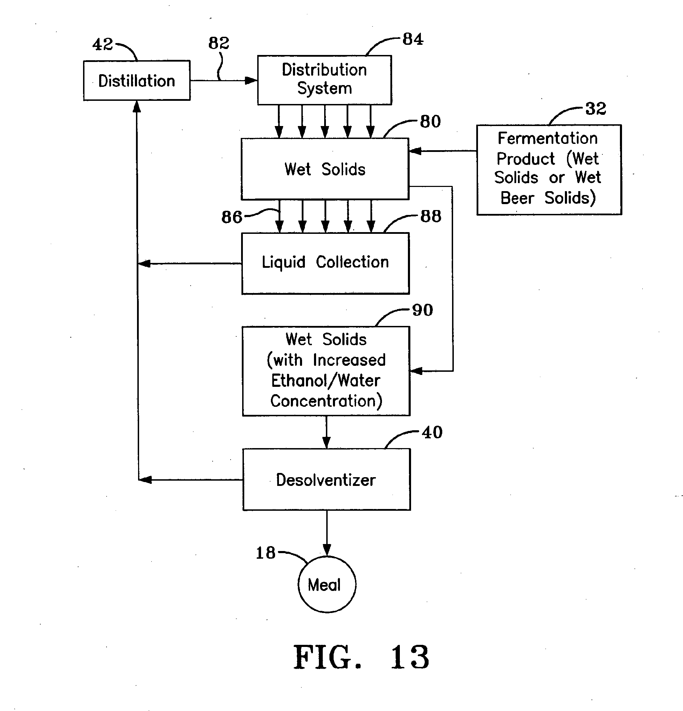

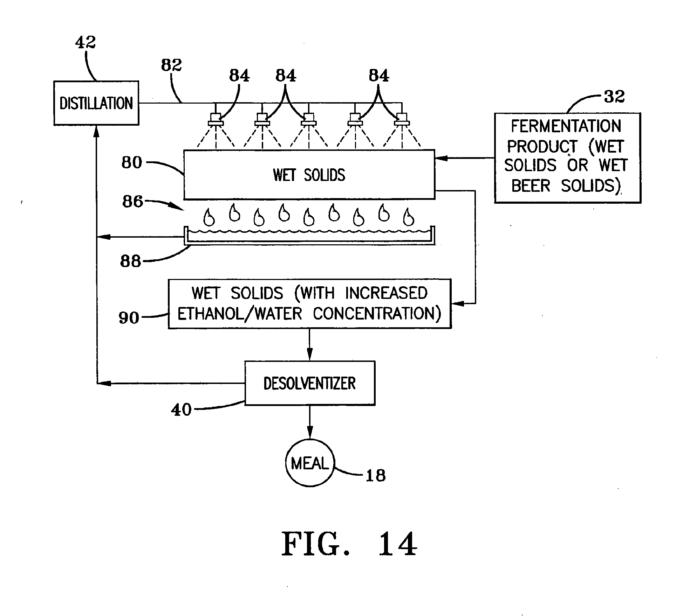

FIGURES 13 and 14 are a block flow diagram and a process flow diagram of an exemplary embodiment of solids processing system 34. Wet solids components 80 (e.g., wet solids 64 or stillage 66) may be washed with ethanol 16 to increase the ethanol concentration, facilitating the reduction of energy required for drying. Wet solids components 80 may generally comprise wet solids or wet beer solids. Washing with ethanol may lead to lower drying energy consumption regardless of whether a liquid component (e.g., liquid 62) and a solids component (e.g., wet solids 64) have been separated by separation process 50. The ethanol wash may also be used with wet solids exiting a distillation process, as in existing plants.

A stream of ethanol 16 may be introduced into wet solids components 80. The stream of ethanol 16 may be received from distillation system 42 or may be received from other sources internal or external to biorefinery 10. The stream of ethanol 16 may be fed through ethanol feed flow lines 82 into an ethanol distribution system 84, which applies (e.g., sprays, mists, drips, deposits, or pours over) ethanol 16 to wet solids components 80 to increase the ethanol concentration of wet solids components 80. Although ethanol distribution system 84 is illustrated as a series of spray nozzles, other means of applying ethanol 16 may be used. For example, ethanol 16 may be poured over wet solids components 80. Wet solids components 80 will remain generally unperturbed by the application of ethanol 16. Once wet solids components 80 have been washed with ethanol 16, liquid 86 (e.g., a mixture of water and ethanol) from wet solids components 80 may be collected by a liquid collection 88, such as a tank or collection tray. Co-products (e.g., zein and xanthophylls) may be extracted from liquid 86, and liquid 86 collected by liquid collection 88 may be directed into distillation system 42 for further processing.

Wet solids components 80 washed with ethanol 16 may be transformed into wet solids components 90, which contain an increased concentration of ethanol 16 to water as compared to the initial wet solids components 80. Less energy may be required by a desolventizer 40 to dry (i.e., desolventize) wet solids components 90 (e.g., after ethanol washing) than would be required by desolventizer 40 to dry (i.e., desolventize) wet solids components 80 (e.g., before ethanol washing). The reduction in energy required to desolventize/dry wet solids components 90 to produce meal 18 may be due at least in part to the fact that wet solids components 90 contain an increased concentration of ethanol 16 to water as compared to wet solids components 80.

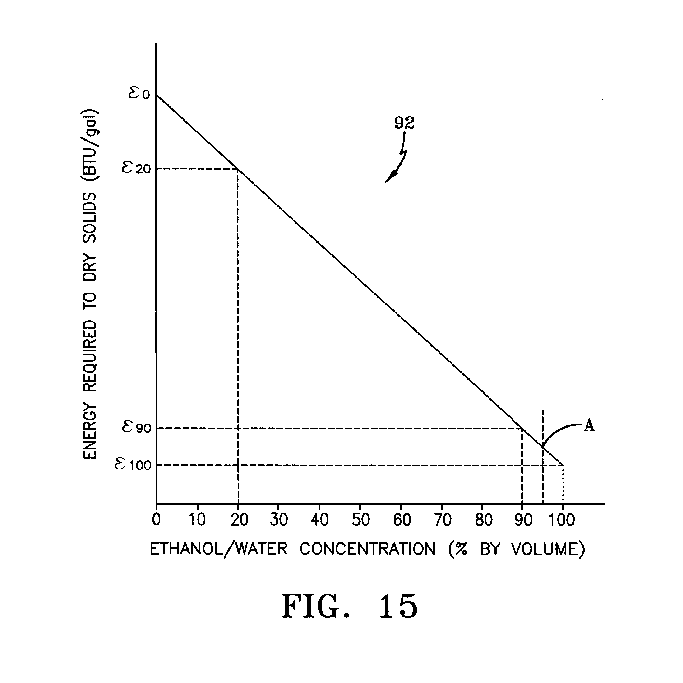

FIGURE 15 is an exemplary graphical representation 92 of energy for drying wet solids as it relates to the amount of ethanol present in the liquid portion of those wet solids. The horizontal axis in FIGURE 15 represents the volume of ethanol present in the liquid portion of the wet solids as a percent of the total liquid volume portion of the wet solids. The vertical axis in FIGURE 15 represents the amount of energy that it would take to dry (e.g., by vaporization) all liquid from wet solids. The graphical representation in FIGURE 15 represents the relationship of the amount of energy to dry the solids within the liquid portion as the concentration of ethanol changes in the liquid portion of the wet solids. In a conventional drying process, the wet solids may contain no ethanol and may require ε₀ amount of energy to remove the liquid from wet solids. By processing the wet solids to contain 20% by volume of ethanol, the amount of energy required to remove the liquid from wet solids would decrease to ε₂₀ as depicted in FIGURE 15. This trend continues as the ethanol concentration is increased, as seen when the ethanol concentration is increased to 90% by volume and the new amount of energy required to remove the liquid portion is drastically reduced to ε₉₀. The lowest amount of energy required to remove the liquid from wet solids can be achieved when the liquid is 100% ethanol.

Returning to FIGURES 13 and 14, increasing the ethanol concentration of wet solids components 90 decreases the amount of energy required to dry wet solids components 90 at least partially because water has a relatively high boiling point, heat capacity, and enthalpy (heat) of vaporization as compared to ethanol. A relatively high amount of energy is required to heat water to a temperature sufficient to vaporize the water; a relatively low amount of energy is required to heat ethanol to a temperature sufficient to vaporize ethanol. For example, the boiling point of ethanol is approximately 173 °F at atmospheric pressure; the boiling point of water is approximately 212 °F at atmospheric pressure. The heat capacity of ethanol is approximately 0.58 BTU/lb-°F; the heat capacity of water is 1.0 BTU/lb-°F. The enthalpy (heat) of vaporization of ethanol is approximately 362 BTU/lb; the enthalpy (heat) of vaporization of water is approximately 980 BTU/lb. Ethanol may be heated with less energy input, reaching its boiling point at a lower temperature, and once at the boiling point, vaporizes with less energy input.

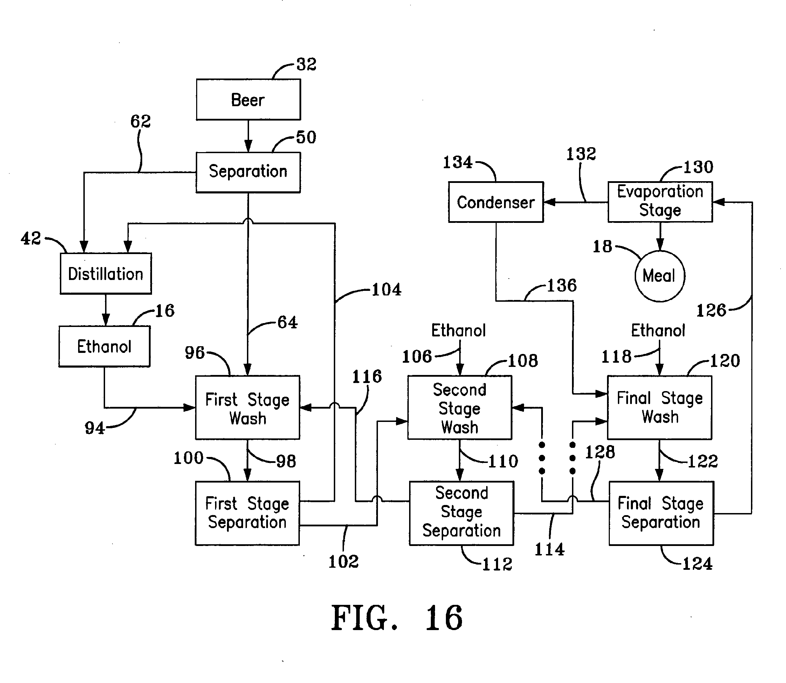

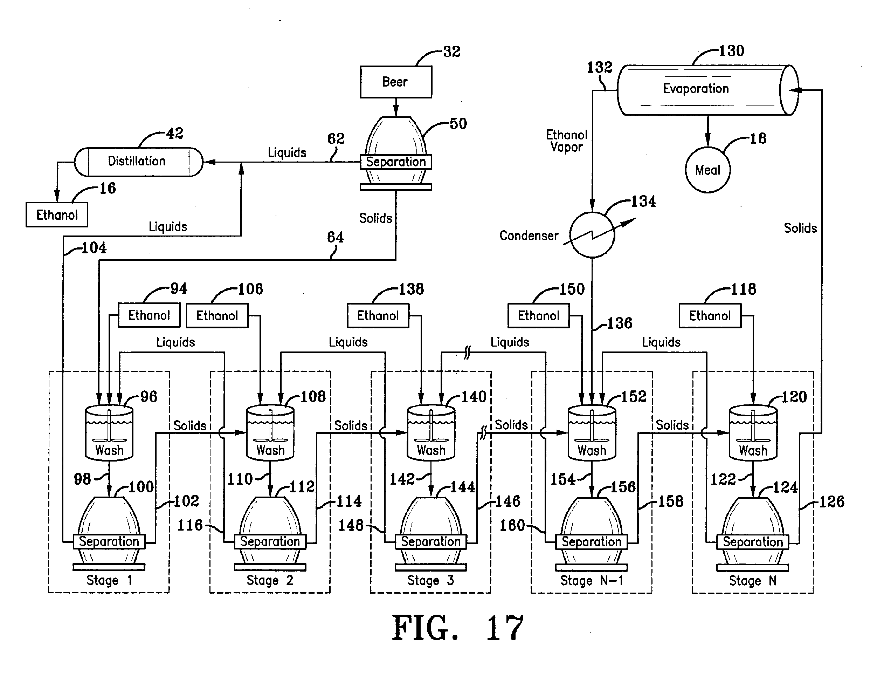

Increasing the ethanol concentration of wet solids components 90 helps to decrease the amount of energy required by desolventizer 40 to dry/desolventize wet solids components 90. FIGURES 13 and 14 include one stage of ethanol washing. FIGURES 16 and 17 are a block flow diagram and a process flow diagram of exemplary embodiments of solids processing system 34, comprising multiple ethanol wash stages. Separation process 50 may separate a liquid component shown as liquid 62 (e.g., a water/ethanol mixture) from a solids component shown as wet solids 64 (e.g., a certain amount of ethanol, a certain amount of water, syrup, particulate matter, and dissolved solids). Liquid 62 may be directed into distillation process 46, which may produce ethanol 16.

Ethanol 94 from distillation system 42 may be directed into a first stage wash 96, which also receives wet solids 64 from separation process 50. Wet solids 64 from separation process 50 are washed with ethanol 94 to increase the ethanol concentration of wet solids 64. It should be noted that when reference is made to "ethanol" in the discussions of the wash process, the fluid used may, and in many cases will, be an ethanol-containing fluid, such as a mixture of water and ethanol. Any other suitable solvent (e.g., hexane) may also be used. The "wash" fluid will generally have a higher concentration of ethanol (or other solvent) than the wet solids receiving the wash, displacing water in the wet solids with ethanol (or other solvent).

First stage wash 96 is configured to mix wet solids 64 with ethanol 94. Ethanol-washed solids 98 from first stage wash 96 may be directed into a first stage separation 100, which separates first stage solids 102 from first stage liquid 104. First stage liquid 104 may consist of a water/ethanol mixture, which may be directed back to distillation system 42 for further processing. First stage liquid 104 washes away a certain amount of the water from wet solids 64. First stage solids 102 output from first stage separation 100 will have a higher ethanol concentration than wet solids 64 input into first stage wash 96.

A second stream of ethanol 106 may be directed into a second stage wash 108, which also receives first stage solids 102 from first stage separation 100. First stage solids 102 from first stage separation 100 may be washed with ethanol 106 to further increase the ethanol concentration of first stage solids 102. Second stage wash 108 may be configured to mix first stage solids 102 with ethanol 106. Ethanol 106 may be received from distillation system 42 or may be received from other processes within biorefinery 10.

Ethanol-washed solids 110 from second stage wash 108 may be directed into a second stage separation 112, which may separate second stage solids 114 from second stage liquid 116. Second stage liquid 116 may consist of a water/ethanol mixture, which may be directed back to first stage wash 96. Second stage liquid 116 may supplement or replace ethanol 94 from distillation system 42 to wash wet solids 64 in first stage wash 96. Second stage liquid 116 washes away a certain amount of the water from first stage solids 102. Second stage solids 114 output from second stage separation 112 will have a higher ethanol concentration than first stage solids 102 input into second stage wash 108.

The ethanol wash cycles (e.g., washing and separating) may be repeated multiple times. A last stream of ethanol 118 may be directed into a final stage wash 120, which also receives the previous stage solids from a previous stage separator. The previous stage solids may be washed with ethanol 118 to further increase the ethanol concentration of the previous stage solids. Final stage wash 120 may be configured to mix the previous stage solids with ethanol 118. Ethanol 118 may be received from distillation system 42 or may be received from other processes within biorefinery 10.

Ethanol-washed solids 122 from final stage wash 120 may be directed into a final stage separation 124, which may separate final stage solids 126 from final stage liquid 128. Final stage liquid 128 will consist of a water/ethanol mixture, which may be directed back to the previous stage wash. Final stage liquid 128 may wash away a certain amount of the water from the previous stage solids. Final stage solids 126 output from final stage separation 124 will have a higher ethanol concentration than the previous stage solids input into final stage wash 120.

Final stage solids 126 may then be directed into an evaporation stage 130, in which the remaining liquid may be evaporated from final stage solids 126, leaving dry or substantially dry meal 18. Ethanol vapor 132 recovered from evaporation stage 130 may be condensed by a condenser 134 and added to the final stage ethanol 118 in the final stage ethanol wash cycle, as shown by line 136. In order to effect the condensation of ethanol vapor 132 from evaporation stage 130, a heat exchanger may be used to recover waste heat from any available source, and direct the heat into evaporation stage 130.

FIGURE 17 is substantially similar to FIGURE 16 with additional wash stages illustrated. A third stream of ethanol 138 may be directed into a third stage wash 140, which also receives second stage solids 114 from second stage separation 112. Second stage solids 114 from second stage separation 112 may be washed with ethanol 138 to further increase the ethanol concentration of second stage solids 114. Third stage wash 140 may be configured to mix second stage solids 114 with ethanol 138. Ethanol 138 may be received from distillation system 42 or may be received from other processes within biorefinery 10.

Ethanol-washed solids 142 from third stage wash 140 may be directed into a third stage separation 144, which may separate third stage solids 146 from third stage liquid 148. Third stage liquid 148 may consist of a water/ethanol mixture, which may be directed back to second stage wash 108. Third stage liquid 148 may supplement or replace ethanol 106 from distillation system 42 to wash first stage solids 102 in second stage wash 108. Third stage liquid 148 washes away a certain amount of the water from second stage solids 114. Third stage solids 146 output from third stage separation 144 will have a higher ethanol concentration than second stage solids 114 input into third stage wash 140.

This process continues with a fourth stream of ethanol 150 being used by a next-to-final stage wash 152 to generate ethanol-washed solids 154, which may be separated by a next-to-final stage separation 156. Similar to the other wash stages, solids 158 from next-to-final stage separation 156 may be directed into final stage wash 120; liquid from next-to-final stage separation 156 may be directed to the previous wash stage.

The ethanol wash stages of FIGURES 16 and 17 may be repeated multiple times such that the wet solids contain a low concentration of water and a high concentration of ethanol. The number of ethanol wash stages may be chosen to achieve a particular concentration of ethanol in the resultant wet solids prior to drying. The concentration of the resultant wet solids prior to drying may be selectively adjusted. In each ethanol wash stage, the washes receive a quantity of ethanol wash containing a higher ethanol concentration than the concentration present in the wet solids. For each ethanol wash stage, a water/ethanol mixture may be received from a subsequent ethanol wash stage as an ethanol wash source. The water/ethanol mixture from a subsequent ethanol wash stage may be suitable for a previous ethanol wash stage because the water/ethanol mixture from the subsequent ethanol wash stage may generally contain more ethanol than the wet solids of the previous ethanol wash stage. Using ethanol from a subsequent ethanol wash stage allows the same stream of ethanol 94 brought into the initial ethanol wash stage to be used over and over again until the final ethanol wash stage. If enough ethanol wash stages are used, the composition of the resulting wet solids will have an ethanol concentration approximately equal to the ethanol concentration of ethanol used in the initial ethanol wash stage. After the multiple ethanol wash stages, the resultant final stage solids 126 contain a liquid component with a higher concentration of ethanol than wet solids 64 from separation process 50. In certain embodiments, ethanol stream 118 may be the only stream of ethanol used; ethanol streams 94, 106, 138, and 150 may be used as make-up ethanol, to selectively control the concentration of ethanol in the other wash stages, or may be omitted.

Returning to FIGURE 15, to minimize the energy to dry meal 18, the ethanol concentration of the resultant final stage solids 126 may be at or above the azeotropic ratio for water and ethanol (e.g., point A), which is approximately 96% ethanol-to-water. At concentration levels at or above the azeotropic ratio, ethanol in wet solids will vaporize at substantially the same rate as the remaining water in the wet solids, leaving meal 18 while using the least amount of energy for drying. With a ratio of ethanol-to-water below the azeotropic ratio, drying of wet solids will be less efficient than when the ratio is at or above the azeotropic ratio. The process may bring the ethanol content of the wet solids to any point along the concentration line with consequent benefits to drying. In an effort to achieve certain benefits, the wet solids will be brought to an ethanol concentration above the azeotropic ratio.

Returning now to FIGURES 16 and 17, evaporation stage 130 may employ a dryer for drying the resultant final stage solids 126, otherwise referred to as wet cake. The drying process may expose meal 18 to temperatures just high enough to vaporize ethanol vapor 132 from meal 18. Meal 18 may be altered and change color when exposed to high temperatures. By limiting the temperature that final stage solids 126 experience by using a low-energy drying process, the possibility of altering the meal 18 during evaporation stage 130 may be substantially reduced. Because final stage solids 126 may be dried at much lower temperatures and with less energy input than in conventional processes, a large volume of air may not be required during evaporation stage 130. Because a lower volume of air may be used in evaporation stage 130 for low-energy drying, the concentration of liquid in the resulting ethanol vapor 132 may be relatively high. Ethanol vapor 132 may also be condensed and re-used within biorefinery 10, reducing emissions from biorefinery 10. Because water used in evaporation stage 130 may be re-circulated, more of the water initially injected into the saccharification (if used) and fermentation processes of biorefinery 10 may be sent back to distillation system 42, where it may be captured and re-used, significantly reducing the overall water consumption of biorefinery 10. The specific equipment of FIGURES 16 and 17 are merely illustrative. Other specific equipment and processes may be used to implement multiple ethanol wash stages to increase the ethanol concentration of wet solids for the purpose of reducing the energy required to dry the wet solids.

Each stage of the solids processing (including steps in the wash/separation process) may be conducted on a single apparatus or (as indicated in FIGS. 16-18, for example) may be conducted on separate apparatus of the same type or including different types of apparatus (e.g., a filter belt system, separator, centrifuge, decanter, etc.); different stages of the processing of the solids component may be conducted on the same or on different/separate apparatus. In the processing of the wet solids (e.g. wet cake), additional operations to the wash/separation process, including operations such as soaking, remixing, slurrying, or other processing of the solids component may be conducted in various sequences, before, during or after washing and separating operations.

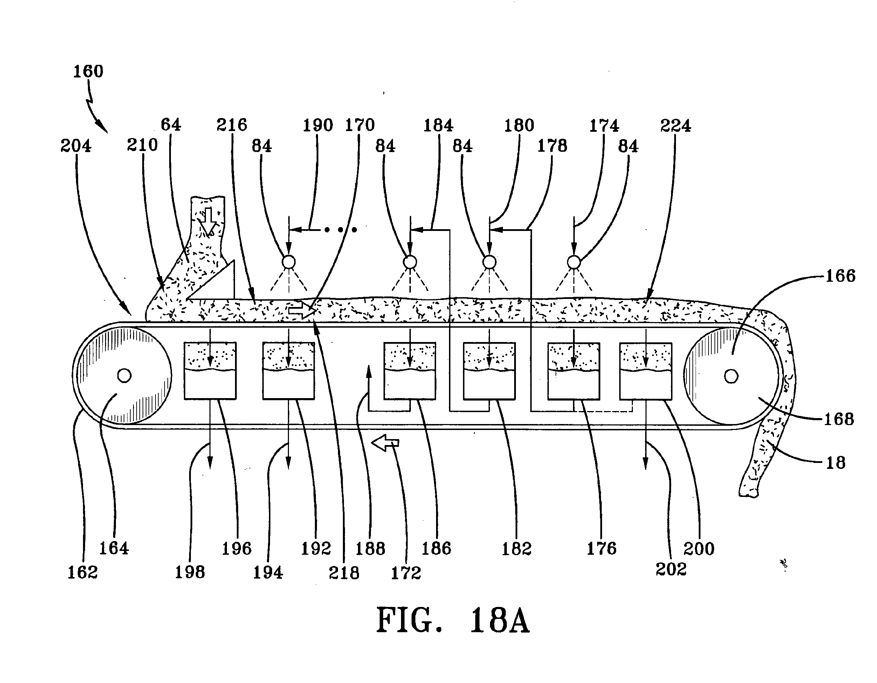

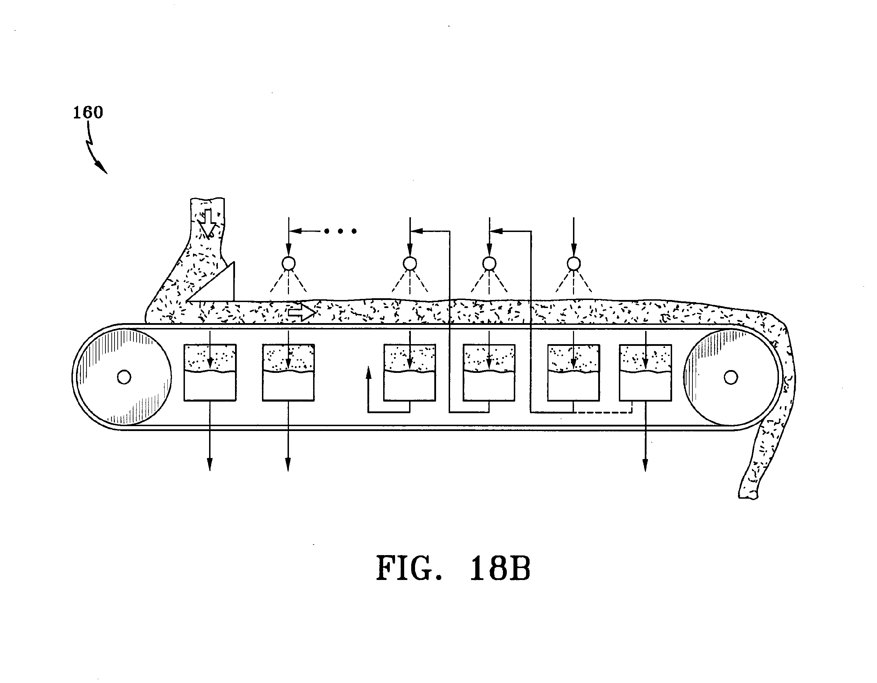

According to an exemplary embodiment, at least a portion of the solids processing can be conducted on a filter belt system shown as including filter belt 162 (see, e.g., FIGURES 18 through 24). The filter belt system may include one or more belts (e.g., for conveying material), some of which may be comprised of a filter media (e.g., to allow the filtration of material on and through the belt), bulk handling systems for loading and unloading the material into and from the system, controls and other instrumentation. The filter belt system may be segmented into stages or chambers (some of which may be configured to operate at differential pressure, including vacuum or positive pressure). According to other embodiments, other combinations of systems may be used for solids processing of the solids component (e.g., wet solids or wet cake).

FIGURES 18A and 18B are cross-sectional views of an exemplary embodiment of an ethanol wash process 160 capable of implementing multiple ethanol wash stages. Ethanol wash process 160 comprises an apparatus shown as a filter belt 162 conveyed by a pair of rollers 164, 166. Rollers 164, 166 are configured to rotate in a clockwise fashion, as shown by arrow 168, causing filter belt 162 to move in a left-to-right direction from the top of roller 164 to the top of roller 166, as shown by arrow 170, and in a right-to-left direction from the bottom of roller 166 to the bottom of roller 164, as shown by arrow 172. The specific relative movement of filter belt 162 and rollers 164, 166 may vary among specific implementations.

Wet solids 64 may be loaded onto the top of filter belt 162 and may move in a left-to-right direction. The left-hand end of filter belt 162 may be referred to as the upstream end; the right-hand end of filter belt 162 may be referred to as the downstream end. Ethanol wash process 160 utilizes a counterflow ethanol wash process. Ethanol may flow through ethanol wash process 160 from the downstream end of filter belt 162 to the upstream end of filter belt 162; wet solids 64 may flow through ethanol wash process 160 from the upstream end of filter belt 162 to the downstream end of filter belt 162. The ethanol used for ethanol wash process 160 generally flows in a direction opposite from the flow of wet solids 64. The ethanol used for ethanol wash process 160 may alternatively flow in the same direction as wet solids 64.

Ethanol may be introduced into ethanol wash process 160 toward the downstream end of filter belt 162. A first stream of ethanol 174 may be applied to (e.g., poured over, sprayed onto, or deposited onto) wet solids 64 by ethanol distribution system 84 above a first downstream collection vessel 176. A mixture of water and ethanol may be drawn through filter belt 162 by gravity and/or vacuum into collection vessel 176 or the flow of the mixture of water and ethanol through filter belt 162 and into collection vessel 176 may be facilitated by positive differential pressure above filter belt 162. The water/ethanol mixture 178 from collection vessel 176 may then be combined with a second stream of ethanol 180, with the combination being applied to (e.g., poured over, sprayed onto, or deposited onto) wet solids 64 by ethanol distribution system 84 above a second downstream collection vessel 182, which is upstream of collection vessel 176. The first stream of ethanol 174 may have a higher, lower, or substantially similar concentration of ethanol than the second stream of ethanol 180. In other embodiments, the second stream of ethanol 180 may not be combined with the water/ethanol mixture 178 from collection vessel 176, but rather only the water/ethanol mixture 178 may be applied to wet solids 64. A mixture of water and ethanol may be drawn through filter belt 162 by gravity and/or vacuum into collection vessel 182 or the flow of the mixture of water and ethanol through filter belt 162 and into collection vessel 182 may be facilitated by positive differential pressure above filter belt 162. The water/ethanol mixture 184 from collection vessel 182 may then be applied to (e.g., poured over, sprayed onto, or deposited onto) wet solids 64 by ethanol distribution system 84 above a third downstream collection vessel 186, which is upstream of collection vessel 182.

Water/ethanol mixture 188 from collection vessel 186 may be directed toward other upstream ethanol distribution systems 84. Ultimately, water/ethanol mixture 190 may be applied to (e.g., poured over, sprayed onto, or deposited onto) wet solids 64 by ethanol distribution system 84 above a first upstream collection vessel 192. Water/ethanol mixture 194 from collection vessel 192 may be directed back to distillation system 42 to recapture some of the ethanol. A second upstream collection vessel 196 upstream of collection vessel 192 may collect water/ethanol mixture 198, which may also be directed back to distillation system 42 to recapture some of the ethanol. A final downstream collection vessel 200 downstream of collection vessel 176 may collect water/ethanol mixture 200, which may also be directed back to distillation system 42 to recapture some of the ethanol. Collection vessel 200 may also act as an evaporation stage, whereby the last remaining water/ethanol mixture 202 may be removed or joined with the water/ethanol mixture 178 from collection vessel 176. A separate drying process, such as evaporation stage 130 of FIGURES 16 and 17 and/or desolventizer 40 of FIGURES 13 and 14 may also be used downstream of ethanol wash process 160.

More or less pure ethanol may be applied to wash the wet solids at the various locations along the filter belt 162. Such concentrations, along with the flow rates of the wash fluid and the speed of the filter belt 162, may serve to control the rate of displacement of water in the wet solids by ethanol. Such factors may regulate the relative difference in ethanol content at each wash stage, as well as the ultimate ethanol content of the wet solids just before final drying.

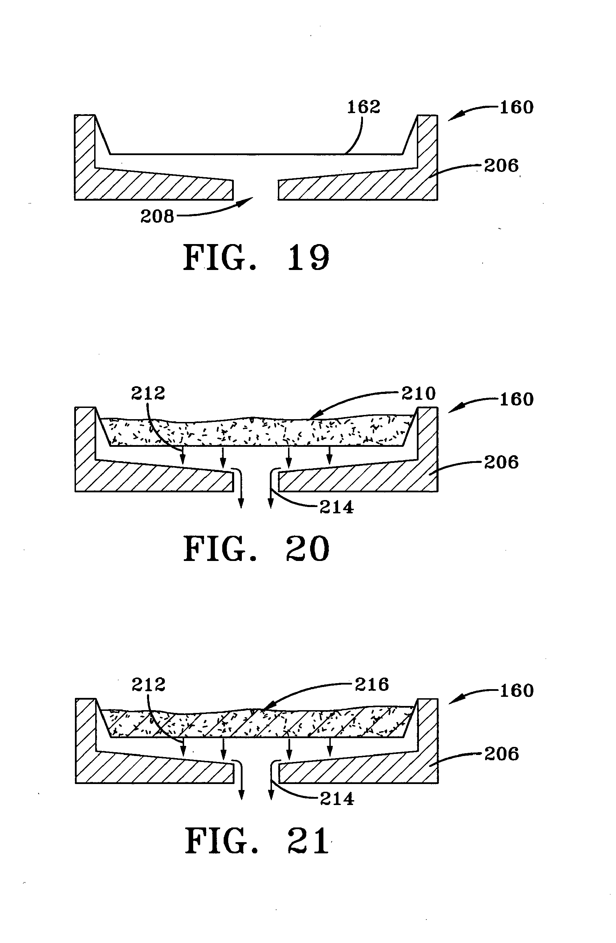

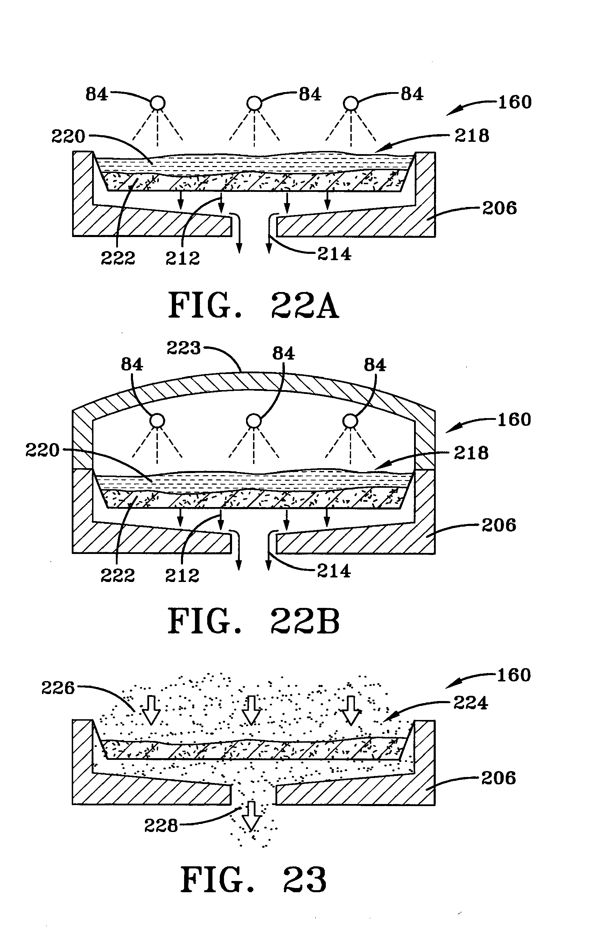

FIGURES 19 through 23 are cross-sectional views at various locations along filter belt 162 of FIGURES 18A and 18B. For example, FIGURE 19 is a cross-sectional view of filter belt 162 of FIGURES 18A and 18B upstream of the location where wet solids 64 are placed onto filter belt 162 shown by arrow 204 in FIGURES 18A and 18B. Although any filter belt technology may be employed, filter belt 162 may have a substantially perforated or porous structure such that a liquid component of wet solids 64 on filter belt 162 may be drawn through filter belt 162 or the flow of the mixture of water and ethanol through filter belt 162 may be facilitated by positive differential pressure above filter belt 162. Filter belt 162 may also include a collection tray 206, which may facilitate the collection of water/ethanol mixtures collected through filter belt 162. Collection tray 206 may include a collection opening 208, through which water/ethanol mixtures may be collected into collection vessels. FIGURE 20 is a cross-sectional view of filter belt 162 of FIGURES 18A and 18B at a location where wet solids 64 are placed onto filter belt 162 shown by arrow 210 of FIGURES 18A and 18B. A vacuum or gravity beneath filter belt 162 may draw a water/ethanol mixture 212 from wet solids 64 onto collection tray 206 and into collection vessel 196, as shown by arrows 214. FIGURE 21 is a cross-sectional view of filter belt 162 of FIGURES 18A and 18B upstream of ethanol distribution system 84 shown by arrow 216. At this point on filter belt 162, wet solids 64 will contain a certain concentration of ethanol. FIGURE 22A is a cross-sectional view of filter belt 162 of FIGURES 18A and 18B at a location near ethanol distribution system 84 shown by arrow 218. At this point, ethanol is introduced into wet solids 64 by ethanol distribution system 84, transforming wet solids 64 into a mixture of liquids 220 (e.g., a water/ethanol mixture) and solids 222. A vacuum or gravity beneath filter belt 162 may draw a water/ethanol mixture 212 from wet solids 64 onto collection tray 206 and into a collection vessel, as shown by arrows 214. FIGURE 22B is a cross-sectional view of filter belt 162 of FIGURES 18A and 18B at a location near ethanol distribution system 84 shown by arrow 218. A pressure chamber 223 may be used to create a differential pressure between the top and bottom of filter belt 162. Pressure may be applied within pressure chamber 223 to facilitate washing by effectively facilitating the flow of the ethanol through wet solids 64. Wet solids 64 downstream of location 218 of filter belt 162 will have a higher ethanol concentration than wet solids 64 upstream of location 218 of filter belt 162. FIGURE 23 is a cross-sectional view of filter belt 162 of FIGURES 18A and 18B at a location near collection vessel 200, as shown by arrow 224. Collection vessel 200 may be used as an evaporation stage. Heated gas 226 may be applied to wet solids 64 above filter belt 162. A vacuum or gravity beneath filter belt 162 may draw heated gas 226 through wet solids 64 and an ethanol vapor 228 may be collected and further processed to recover ethanol and other desirable components present in ethanol vapor 228.

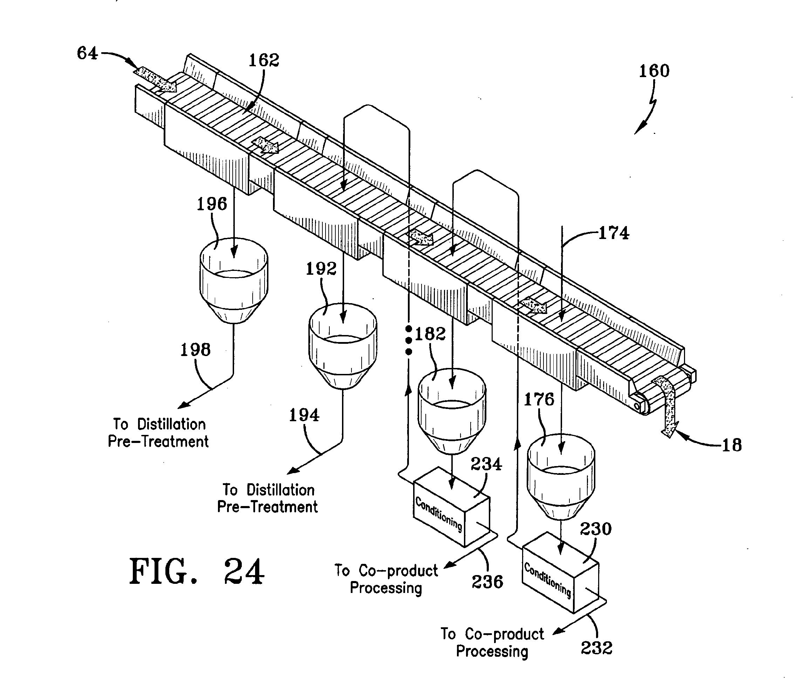

FIGURE 24 is a perspective view of another exemplary embodiment of ethanol wash process 160. Wet solids 64 may be placed on filter belt 162 at an upstream location with meal 18 exiting at a downstream location of filter belt 162. An initial collection vessel 196 may collect water/ethanol mixture 198 without washing, which may be directed back to distillation pre-treatment process 44. The first stream of ethanol 174 may be applied to wet solids 64 above collection vessel 176. A mixture of water and ethanol may be drawn through filter belt 162 by gravity and/or vacuum into collection vessel 176 or the flow of the mixture of water and ethanol through filter belt 162 and into collection vessel 176 may be facilitated by positive differential pressure above filter belt 162. The water/ethanol mixture from collection vessel 176 may be directed into a first conditioning process 230, which may filter and condition the water/ethanol mixture. A portion of the ethanol/water mixture may be directed to co-product processing, as shown by arrow 232; filtered/conditioned ethanol from conditioning process 230 may be applied to wet solids 64 above collection vessel 182.

A mixture of water and ethanol may be drawn through filter belt 162 by gravity and/or vacuum into collection vessel 182 or the flow of the mixture of water and ethanol through filter belt 162 and into collection vessel 182 may be facilitated by positive differential pressure above filter belt 162. The water/ethanol mixture from collection vessel 182 may be directed into a second conditioning process 234, which may filter and condition the water/ethanol mixture. A portion of the ethanol/water mixture may be directed to co-product processing, as shown by arrow 236; filtered/conditioned ethanol from conditioning process 234 may be applied to wet solids 64 above previous upstream collection vessels. Ultimately, a mixture of water and ethanol may be drawn through filter belt 162 by gravity and/or vacuum into collection vessel 192 or the flow of the mixture of water and ethanol through filter belt 162 and into collection vessel 192 may be facilitated by positive differential pressure above filter belt 162. The water/ethanol mixture 194 from collection vessel 192 may be directed to distillation pre-treatment process 44.

In certain embodiments, the apparatus comprising a filter belt may include two or more such belts. For example, a first belt may be used for a first stage of washing with ethanol (or another solvent), while further filter belts may be used for subsequent stages. The material conveyed with the belts may be transferred from one belt to the other as the process progresses. Some embodiments may include intermediate equipment between filter belts, such as mixers for creating a slurry with the solvent or solvent mixture, centrifuges or other separators for performing some degree of moisture removal, and so forth. The filter belts themselves may be of any suitable type, including arrangements in which a semi-permeable belt serves as a substrate used to receive the solids component, and apparatuses with multiple layers of belts, support structures, and so forth.

Meal 18 produced by biorefinery 10 may also be reconstituted into feed at a desired composition to differentiate the product for various markets. For example, some protein and any extracted biochemicals or other bioproducts may be re-applied to the resulting meal to constitute the desired end product. Levels of protein and other amino acids could be selectively adjusted. For example, proteins, fats, syrup, oils, lutein, lysine, zein, and other bioproducts and biochemicals may be selectively combined with the meal. According to preferred embodiment, processing of the meal may take place at temperatures that avoid degradation of the meal itself. The use of such reduced temperatures at all stages of processing (through the plant) results in a meal that is of a different quality than conventional DDG (i.e., resulting from a process employing "cooked" liquefaction). For example, maintaining the processing temperatures below about 150 °C is believed to produce a product that is quite distinct from conventionally processed DDG, and even lower temperatures, on the order of 100 °C (or even lower, at 93 °C, 180 °F, or 130 °F) are particularly helpful in creating a unique meal.

Depending upon the processing, the meal may be referred to as "corn meal" (particularly when corn is the feedstock), "distillers meal", "distillers dried meal", "dried distillers meal", "protein-containing meal", and "corn distillers meal", among others. When the solids component is subject to the distillation process, the resulting product may be different still, somewhat more akin to conventional DDG, although certain benefits of the processing are nevertheless realized, such as the reduction in energy utilization in the biorefinery.

The meal may also be further transformed for particular product categories and markets. For example, the meal may be mechanically pressed or extruded into pellets configured for packaging, transportation, durability, and digestibility. Such processing may be well suited for producing animal feeds. Within this category of product, a number of varieties may be formulated including different ingredients, protein qualities, additives, sizes, and configurations.

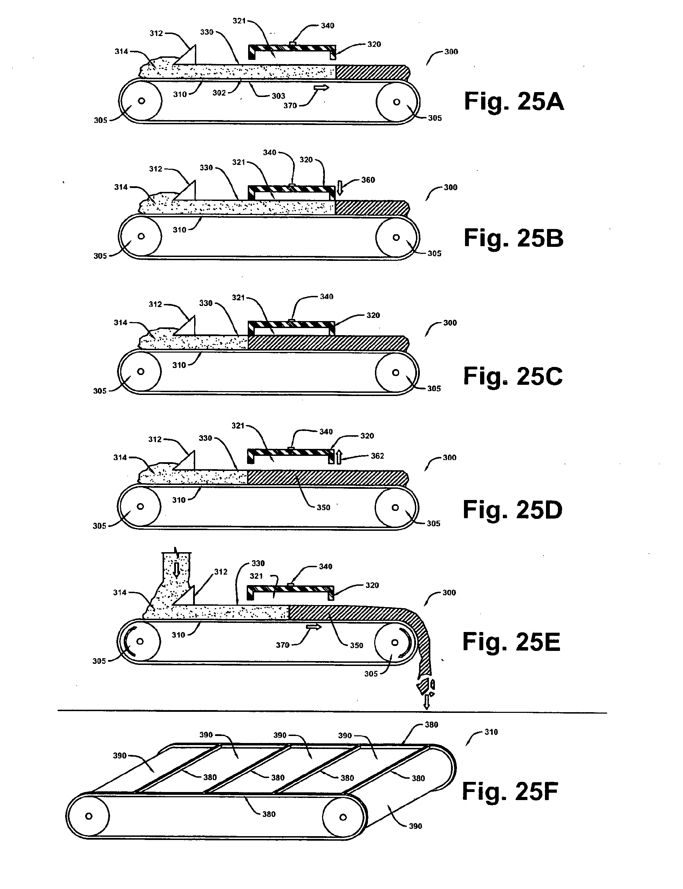

In some embodiments, multiple treatments of a wet solid (e.g., multiple ethanol wash stages) may be performed in a single chamber at a fixed location on a belt that is configured for indexing movement. FIGURES 25A through 25E are schematic cross-sectional views of exemplary embodiments of a wash apparatus 300 and corresponding process that is capable of implementing multiple stage, single chamber washing. Although an exemplary ethanol wash process is described herein, it is understood that the apparatus and process may be employed for other washing and/or treatment processes, for example, the treatment of the wet solids with an additive (e.g., a vitamin). Apparatus 300 includes porous filter belt 310 having first major surface 302 and second major surface 303. Filter belt 310 includes a plurality of pores (not shown) that extend between first major surface 302 and 303 to provide the filter belt 310 with porosity to liquids. The filter belt 310 is mounted on rollers 305 that can be advanced by a drive mechanism (not shown) to move the filter belt 310 in indexing movements (i.e., starting and stopping between positions) in the downweb direction shown by arrow 370. Apparatus 300 further includes coating apparatus 312 that applies wet solids 314 to the first major surface 302 of filter belt 310 in the form of a layer of wet solids 330. Apparatus 300 also includes wash chamber 320 having cavity 321 that is configured for movement between a first position (see, FIGURE 25A) where the wash chamber 320 is separated from the filter belt 310 and a second position (see, FIGURE 25B) where the wash chamber 320 is in sealing contact with either the layer of wet solids 330 or the first major surface 302 of the filter belt 310.

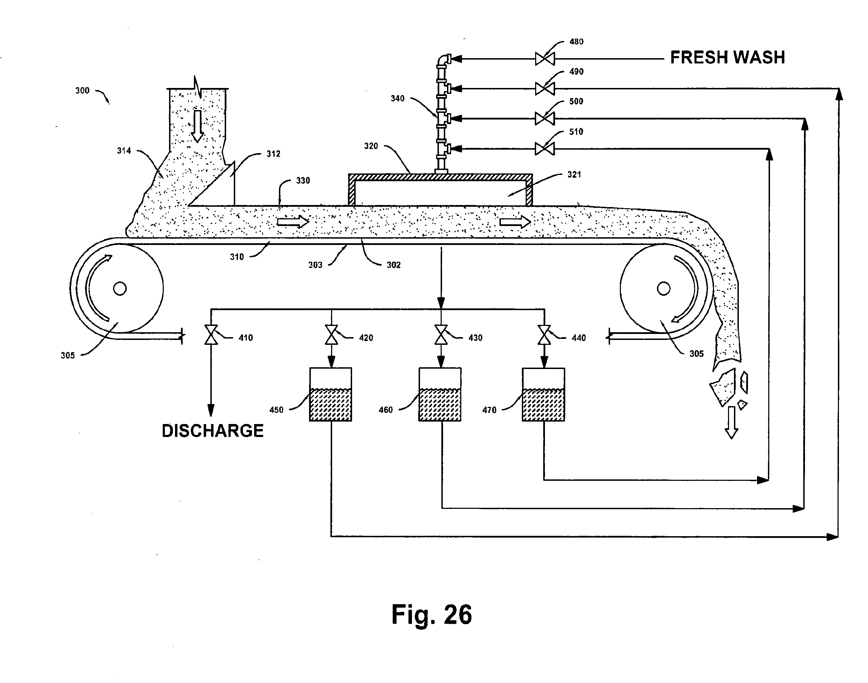

As detailed in FIGURE 25A through FIGURE 25F, multiple wash stages of an ethanol wash process may be carried out in chamber 320 at a fixed location on an indexing-style filter belt 310 (e.g., a filter belt that starts and stops between positions). FIGURES 25A through 25E and FIGURE 26 illustrate the operation of an exemplary ethanol wash cycle using a indexing-style filter belt 300 and detail how chamber 320 (e.g., an application chamber) may be brought into contact with wet solids 330 for the purpose of washing the wet solids with one or more liquids.

Referring now to FIGURE 25A, the start of the process is shown. In FIGURE 25A, the filter belt 310 is in a position for chamber 320 to be lowered into sealing contact with the layer of wet solids 330. FIGURE 25B illustrates the chamber 320 after being placed into contact with the layer of wet solids 330 by movement of chamber 320 in direction 360. FIGURE 25C illustrates the ethanol washing sequence, as further described in more detail hereinbelow and with reference to FIGURE 26. During the wash process, one or more liquids are provided to the chamber 320 through orifice 340 as more fully described with reference to FIGURE 26. The interior of the chamber includes one or more nozzles (not shown) for applying the liquid to the wet solids. The one or more liquids flow through the layer of wet solids and porous filter belt optionally with the aid of pressure, heat, and/or vacuum) and may be recovered for reuse in the process. Referring now to FIGURE 25D, after the ethanol wash sequence is completed, the chamber 320 is raised in the direction of arrow 362, as illustrated in FIGURE 25D. As illustrated in FIGURE 25D, the washed solids 350 below the chamber 320 has completed a stage of ethanol washing, as indicated by the hatched area 350. Referring now to FIGURE 25E, after raising chamber 320, the filter belt 310 is indexed in the direction shown by arrow 370 to move the washed solids 350 from under the chamber 320. After indexing, the ethanol wash cycle may be repeated as shown in FIGURE 25A.

FIGURE 26 is a schematic cross-sectional view of the ethanol wash process 300 of FIGURES 25A through 25F during the ethanol washing step of FIGURE 25C. A counter-current ethanol wash process may be accomplished by first opening valve 410. Valve 490 may then be opened to allow the water/ethanol mixture from tank 450 to flow through chamber 320, which is sealed to the layer of wet solids 330 being washed on the filter belt 310. Tank 450 may be pressurized or a pump may be used to facilitate the flow of the water/ethanol mixture from tank 450 into the chamber 320 and through the wet solids layer 330. Filter belt 310 is porous to the flow of liquid thereby allowing the water/ethanol mixture to pass through the wet solids and filter belt, optionally with the aid of heat, vacuum, and/or pressure. After the wet solids have been washed with the water/ethanol mixture from tank 450 it is discharged through valve 410.

After washing the wet solids 330 with the water/ethanol liquid from tank 450, both valves 410 and 490 may be closed. Next, valves 420 and 500 may be opened such that the water/ethanol mixture from tank 460 may be used as the next ethanol wash fluid. Tank 460 may be pressurized or a pump may be used to facilitate the flow of the water/ethanol mixture from tank 460 into the chamber 320 and through the layer of wet solids 330. Filter belt 310 is porous to the flow of liquid thereby allowing the water/ethanol mixture to pass through the wet solids and porous filter belt optionally with the aid of heat, vacuum, and/or pressure. After flowing through the wet solids 330, the water/ethanol mixture from tank 460 flows into tank 450 where it is retained to be used in the next cycle of the process (i.e., after the next indexing of the filter belt).

After the wet solids 330 have been washed with the water/ethanol mixture from tank 460, both valves 420 and 500 may be closed. Next, valves 430 and 510 may be opened such that the water/ethanol mixture from tank 470 may be used as the next wash liquid. Tank 470 may be pressurized or a pump may be used to facilitate the flow of the water/ethanol mixture from tank 470 into the chamber and through the wet solids 330. Filter belt 310 is porous to the flow of liquid thereby allowing the liquid to pass through the wet solids and porous filter belt optionally with the aid of heat, vacuum, and/or pressure. After flowing through the layer of wet solids 330, the water/ethanol mixture from tank 470 flows into tank 460 where it is retained to be used in the next cycle of the process (i.e., after the next indexing of the filter belt).

After the wet solids 330 have been washed with the water/ethanol mixture from tank 470, both valves 430 and 510 may be closed. Next, valves 440 and 480 may be opened such that a fresh ethanol wash (e.g., having a high concentration of ethanol and a relatively low concentration of water) may be used as the ethanol wash fluid. The fresh ethanol wash may flow through the chamber 320 where it is applied to the layer of wet solids 330. The fresh ethanol wash then flows through porous filter belt 310 and is recovered and stored in tank 470, where it is held to be used in the next cycle of the process (i.e., after the next indexing of the filter belt). After the wet solids 330 have been washed with the fresh ethanol wash, both valves 440 and 480 may be closed. After treatment of the wet solids 330, the chamber 320 can be lifted and the belt 310 indexed as shown in FIGURE 25D and FIGURE 25E.

The steps of implementing a counter-current ethanol wash process are merely exemplary and not intended to be limiting. Indeed, the same type of sequence may use more (e.g., 5, 6, 7, 8, 9, or 10) or less (e.g., 2 or 3) washes for a give index of the filter belt 310.

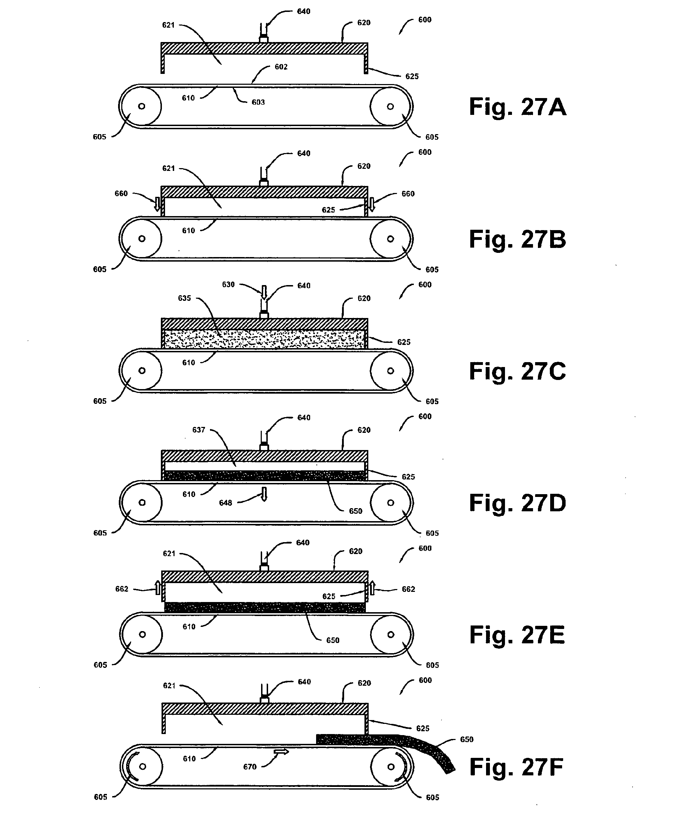

Referring now to FIGURES 27A through 27F, a schematic cross-sectional view of an exemplary embodiment of a single chamber ethanol wash process 600 is shown. The single chamber system of FIGURES 27A through 27F may have an advantage of not requiring the chamber 620 to be sealed to the wet solids 630, but may instead seal the chamber 620 directly to the first major surface 602 of filter belt 610. As previously described, filter belt 610 is porous and includes a plurality of pores (not shown) for allowing liquid to move from the first major surface 602 to the second major surface 603. The chamber 620 of FIGURES 27A through 27F contains a lower housing 625 defining cavity 621. As shown in FIGURE 27B, the lower housing 625 may be lowered into place on the stationary filter belt 610 by moving the chamber 620 in the direction shown by arrows 660. After the chamber 620 has been moved into place, the wet solids 630 may be dispensed through orifice 640 to at least partially fill closed cavity 635 that is formed between chamber 620 and filter belt 610, as illustrated in FIGURE 27C. Excess liquid may then be removed through filter belt 610 by applying vacuum 648 to the second major surface 603 of filter belt 610. Optionally, the cavity 635 may be pressurized and/or heat may be applied to the wet solids 630 to aid in removal of the liquid. Removal of the liquid causes cake 650 to build up within the cavity 635, as illustrated in FIGURE 27D. While in the state illustrated in FIGURE 27D, multiple ethanol washes may be performed, for example, using a counter-current process as described herein with respect to FIGURE 26. After the number of desired ethanol washes have been performed using desired ethanol wash pressures, the chamber 620 may be raised by being moved in the direction of arrow 662, as illustrated in FIGURE 27E. Once the chamber 620 is raised, leaving the cake 650 on the filter belt 610, the filter belt 610 may be indexed in a downweb direction 670 to discharge the cake 650 at the end of the filter belt 610, as illustrated in FIGURE 27F. Once the indexing is completed, the ethanol wash cycle may be repeated again by repeating the steps of FIGURES 27B to 27F.

According to an exemplary embodiment, the open space 637 that is present above the cake 650 in cavity 635 (see, FIGURE 27D) may be closed or partially closed. A top portion of the chamber 620 may be capable of moving so that it comes into contact, or nearly comes into contact, with the cake 650 prior to applying the ethanol wash fluid. This may act to reduce liquid retention above the cake 650, reduce the ethanol wash time, improve distribution of the wet solids, and/or reduce the risk of cross-contamination of the ethanol wash fluids.

Referring now to FIGURE 25F, in treatment processes where the chamber comes into contact with the wet solids, a certain amount of the wet solids that are positioned under the edges of the chamber may not receive the volume of the wash that was desired. In view of this, in some embodiments, a filter belt as shown in FIGURE 25F may be advantageously used. Filter belt 310 of FIGURE 25F includes raised portions 380 (e.g., ribs) that are raised above the first major surface 390 of filter belt 310. The raised portions 380 are arranged to match up with the chamber such that the chamber walls come into contact with the raised portions 380 of the filter belt 310 (i.e., as opposed to coming into direct contact with the wet solids). In this way, a seal between the chamber and the filter belt is formed thereby avoiding direct contact between the chamber walls and the wet solids. The raised portions 380 may be molded directly into the filter belt 310 or they may be manufactured as one or more separate pieces that are attached to the first major surface 390 of the filter belt 310.

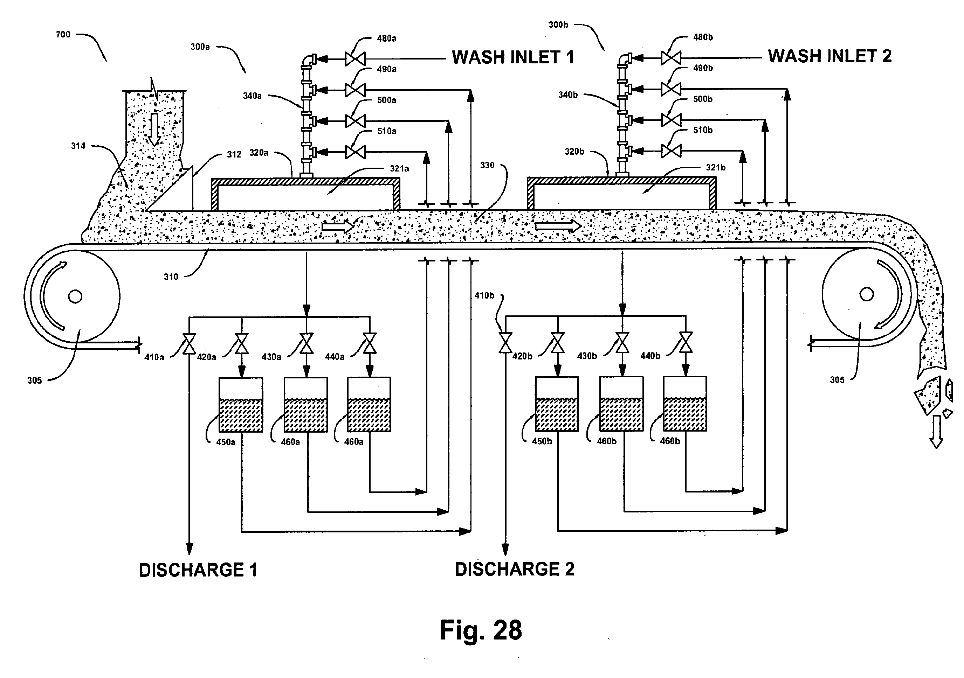

In some embodiments, more than one chamber may be used on a single filter belt in order to treat wet solids. The chambers may be arranged parallel (i.e., crossweb) relative to each other, in series (i.e., downweb) relative to each other, or they may be arranged in a combination of parallel and serial arrangement. The two or more chambers may be separate and capable of being moved independently of one another (see, e.g., FIGURE 28) or a single movable chamber may comprise multiple sub-chambers with each sub-chamber being configured to separately treat the wet solids.

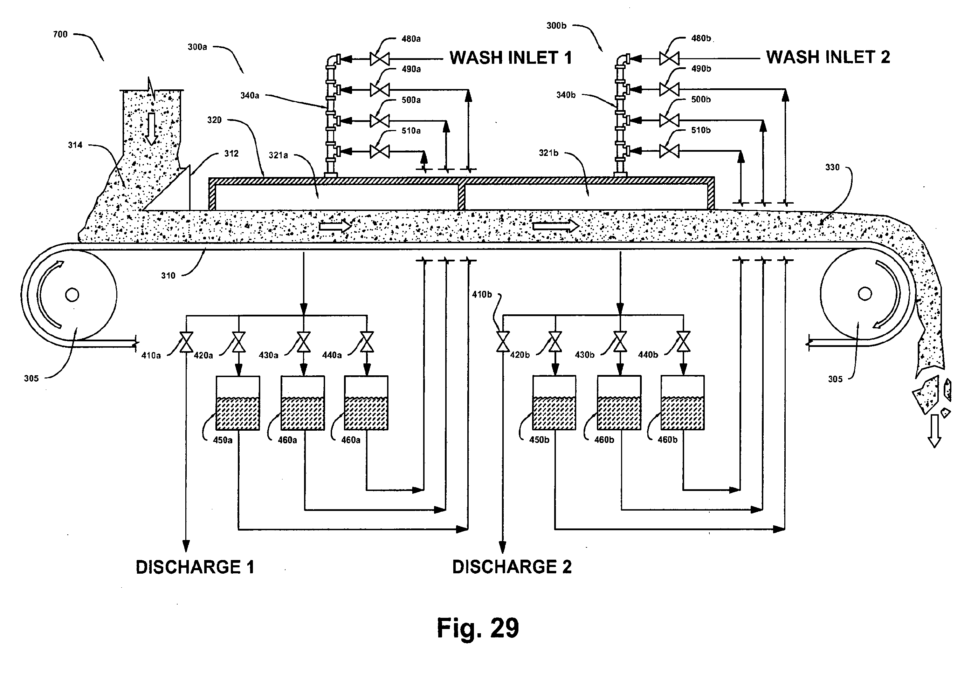

Referring now to FIGURES 28-29, embodiments of multi-chamber treatment apparatuses and processes 700 are shown. Each apparatus 700 includes two separate wash apparatuses 300a and 300b that are arranged in series (i.e., downweb) on filter belt 310. In FIGURE 28, the apparatus and process includes application chambers 320a and 320b (defining cavities 321a and 321b) that are configured for independent movement relative to the filter belt (i.e., movement into an out of contact with the filter belt or wet solid). In FIGURE 29, the apparatus includes chamber 320 that comprises sub-chambers 320a and 320b. Sub-chamber 320a defines cavity 321a and sub-chamber 320b defines cavity 321b. In the apparatus and process of FIGURE 29, the sub-chambers 320a and 320b move together relative to the filter belt. The operation of each wash apparatus 300a and 300b may be conducted independently as described, for example, hereinabove with reference to FIGURE 26. Two or more wash apparatuses that are arranged in series provide an advantage because multiple treatments may be conducted simultaneously along a single filter belt. For example, a first treatment may be conducted in chamber 320a while a different treatment is conducted simultaneously in chamber 320b. One advantage of this process is that two or more shorter-duration treatments can be conducted at the same time as one or more longer-duration treatments. For example, if the treatment that takes place in wash apparatus 320a takes about 1 minute to complete, multiple shorter-duration treatments (e.g., 2 or more) may be conducted in wash apparatus 320b during the time period needed for the longer-duration treatment in chamber 320a. For example, three separate treatments totaling about 1 minute (e.g., 3 - 20 second treatments) in duration could be conducted in chamber 320b while a treatment of about 1 minute in duration is conducted in chamber 320a. The ability to "stack" multiple shorter treatments may be desirable, for example, in instances where the properties and/or composition of the material that is being treated changes during the process thereby modifying the desired time duration of the treatments. For example, if the porosity of the material changes as a result of a first treatment of a material, then it may be possible to increase the number of treatments that can subsequently take place in a treatment period in view of the increased porosity of the material. Treatment using multiple sealed chambers may also be advantageous in order to separate incompatible treatment steps from one another or to provide different environments (e.g., heat, pressure, etc.) for treatment within the same process. In addition to washing the solids, useful treatments may include, for example, the addition of a property or performance enhancing additive to the washed solids. For example, the addition of a vitamin, color, flavor, digestibility aid, or processing aid (e.g., such as surface tension treatments) to improved end user processing, may be desirable.

## Claims

1. An apparatus for treating a layer of wet solids obtained from a fermentation process (330, 630) with one or more liquids, the apparatus comprising:
(a) a belt (162, 310) having a first major surface (302) and a second major surface (303) and a plurality of pores extending between the first major surface (302) and the second major surface (303) to provide the belt (162, 310) with porosity to the one or more liquids;
(b) a drive system for advancing the belt (162, 310) in indexing movements in a downweb direction;
(c) a treatment chamber (320, 620) configured for movement between:
(i) a first position where the treatment chamber (320, 620) is separated from the belt (162, 310) in order to allow the belt (162, 310) to be advanced by the drive system in indexing movements in a downweb direction; and
(ii) a second position where the treatment chamber (320, 620) is in sealing contact with the first major surface (302) of the belt (162, 310) or with the layer of particulate solids positioned on the first major surface (302) of the belt (162, 310);
(d) one or more nozzles in the treatment chamber (320, 620) for applying one or more liquids to the wet solids (330, 630); and
(e) one or more reservoirs (176, 182, 186, 192, 196, 450, 460, 470) for holding one or more liquids for treatment of the wet solids (330, 630); wherein the one or more reservoirs (176, 182, 186, 192, 196, 450, 460, 470) are in fluid communication with the one or more nozzles.

2. The apparatus of claim 1,further including a vacuum system (648) positioned proximate the second major surface (303) of the belt (162, 310) for removing at least a portion of liquid applied to the wet solids (330, 630) through the pores in the belt (162, 310) to form an effluent.

3. The apparatus of claim 1, wherein the belt (162, 310) includes raised portions (380) that are arranged in a pattern on the first major surface (302) of the belt (162, 310) so that they mate with the treatment chamber (320, 620) to form a seal between the belt (162, 310) and the treatment chamber (320, 620).

4. The apparatus of claim 3, wherein the raised portions (380) are molded into the belt (162, 310).

5. The apparatus of claim 1, wherein the apparatus comprises two or more treatment chambers (320, 620) arranged in series in a downweb direction over the belt (162, 310).

6. The apparatus of claim 1, wherein the apparatus comprises a treatment chamber (320, 620) comprising two or more sub-chambers (320a, 320b).

7. The apparatus of claim 1, wherein the treatment chamber (320, 620) can be pressurized when in sealing contact in order to increase flow of liquid through the layer of wet solids (330, 630) and the belt (162, 310).

8. The apparatus of claim 1, wherein the apparatus comprises two or more reservoirs (176, 182,186,192,196,450,460,470).

9. A method of treating wet solids obtained from a fermentation process with one or more liquids to form a treated solid, the method comprising the steps of:
(a) providing an apparatus according to claim 1;
(b) applying wet solids to the first major surface of the belt to form a layer of wet solids;
(c) moving the treatment chamber from (i) a first position where the treatment chamber is separated from the belt to (ii) a second position where the treatment chamber is in sealing contact with the first major surface of the belt or with the layer of wet solids;
(d) applying one or more liquids to the layer of wet solids positioned under the treatment chamber while the treatment chamber is in sealing contact; and
(e) at least partially removing the one or more liquids from the wet solids to form an effluent.

10. The method of claim 9, wherein the step (d) comprises sequentially applying two or more liquids to the wet solids.

11. The method of claim 9, wherein step (e) comprises applying vacuum to the second major surface of the belt.

12. The method of claim 9, wherein step (d) is conducted under pressure in the treatment chamber and/or wherein step (e) is conducted without the use of vacuum and/or wherein, after step (e), the method further includes the step of moving the treatment chamber from the second position to the first position and advancing the belt a distance in the downweb direction.

13. A method of treating wet solids from a fermentation process with one or more liquids to form a treated solid, the method comprising the steps of:
(a) providing an apparatus according to claim 5; wherein the apparatus comprises a first treatment chamber and a second treatment chamber;
(b) applying wet solids to the first major surface of the belt to form a layer of wet solids;
(c) moving the first treatment chamber and the second treatment chamber into sealing contact with the first major surface of the belt or with the layer of wet solids;
(d) applying a first liquid to the layer of wet solids under the first treatment chamber;
(e) sequentially applying two or more liquids to the layer of wet solids under the second treatment chamber;
(f) at least partially removing at least a portion of the liquids applied in steps (d) and (e) from the wet solids by applying vacuum to the second major surface of the belt;
(g) moving the first and second treatment chambers out of sealing contact with the belt; and
(h) advancing the belt a distance in the downweb direction.

14. The method of claim 13, wherein steps (b) to (h) are repeated to provide a continuous treatment process.

15. The method of claim 13, wherein steps (d), (e), and (f) are performed during approximately the same time period so that upon completion of step (f) the wet solids under the first treatment chamber have been treated by the first liquid, and the wet solids under the second treatment chamber have been treated by the two or more liquids.

16. The method of claim 9 or claim 13, wherein at least one of the liquids comprises a mixture of ethanol and water.

17. The method of claim 9 or claim 13, wherein at least one of the liquids comprises an additive for treatment of the wet solids, such as a vitamin, color, flavor, digestibility aid, or downstream processing aid.

18. The method of claim 9 or claim 13, wherein the treatment displaces water with ethanol and/or wherein the treatment is conducted without raising the temperature of the wet solids above a temperature of about 150° C, such as about 65°C.

## Patentansprüche

1. Vorrichtung zum Behandeln einer Schicht von nassen Feststoffen, die von einem Fermentationsprozess (330, 630) mit einer oder mehreren Flüssigkeiten erhalten wurden, wobei die Vorrichtung Folgendes umfasst:
(a) ein Band (162, 310) mit einer ersten Hauptfläche (302) und einer zweiten Hauptfläche (303) und mehreren Poren, die zwischen der ersten Hauptfläche (302) und der zweiten Hauptfläche (303) verlaufen, um dem Band (162, 310) Porosität für die eine oder mehreren Flüssigkeiten zu verleihen;
(b) ein Antriebssystem zum Vorwärtsbewegen des Bandes (162, 310) in Schaltbewegungen in einer Bahnabwärtsrichtung;
(c) eine Behandlungskammer (320, 620), konfiguriert für eine Bewegung zwischen:
(i) einer ersten Position, in der die Behandlungskammer (320, 620) von dem Band (162, 310) getrennt ist, um es zuzulassen, dass das Band (162, 310) vom Antriebssystem in Schaltbewegungen in Bahnabwärtsrichtung vorwärtsbewegt wird; und
(ii) einer zweiten Position, in der die Behandlungskammer (320, 620) in einem Dichtungskontakt mit der ersten Hauptfläche (302) des Bandes (162, 310) oder mit der Schicht von partikelförmigen Feststoffen ist, die sich auf der ersten Hauptfläche (302) des Bandes (162, 310) befindet;
(d) eine oder mehrere Düsen in der Behandlungskammer (320, 620) zum Aufbringen von einer oder mehreren Flüssigkeiten auf die nassen Feststoffe (330, 630); und
(e) einen oder mehrere Vorratsbehälter (176, 182, 186, 192, 196, 450, 460, 470) zum Aufnehmen von einer oder mehreren Flüssigkeiten zum Behandeln der nassen Feststoffe (330, 630); wobei die ein oder mehreren Vorratsbehälter (176, 182, 186, 192, 196, 450, 460, 470) in Fluidverbindung mit den ein oder mehreren Düsen sind.

2. Vorrichtung nach Anspruch 1, die ferner ein Unterdrucksystem (648) umfasst, das in der Nähe der zweiten Hauptfläche (303) des Bandes (162, 310) positioniert ist, um wenigstens einen Teil von auf die nassen Feststoffe (330, 630) aufgebrachter Flüssigkeit durch die Poren in dem Band (162, 310) zu entfernen, um einen Abfluss zu bilden.

3. Vorrichtung nach Anspruch 1, wobei das Band (162, 310) erhabene Abschnitte (380) aufweist, die in einem Muster auf der ersten Hauptfläche (302) des Bandes (162, 310) angeordnet sind, so dass sie sich mit der Behandlungskammer (320, 620) paaren, um eine Dichtung zwischen dem Band (162, 310) und der Behandlungskammer (320, 620) zu bilden.

4. Vorrichtung nach Anspruch 3, wobei die erhabenen Abschnitte (380) in das Band (162, 310) eingeformt sind.

5. Vorrichtung nach Anspruch 1, wobei die Vorrichtung zwei oder mehrere Behandlungskammern (320, 620) umfasst, die in Reihe in einer Bahnabwärtsrichtung über dem Band (162, 310) angeordnet sind.

6. Vorrichtung nach Anspruch 1, wobei die Vorrichtung eine Behandlungskammer (320, 620) umfasst, die zwei oder mehrere Teilkammern (320a, 320b) umfasst.

7. Vorrichtung nach Anspruch 1, wobei die Behandlungskammer (320, 620) unter Druck gesetzt werden kann, wenn sie in Dichtungskontakt ist, um den Fluss von Flüssigkeit durch die Schicht von nassen Feststoffen (330, 630) und das Band (162, 310) zu erhöhen.

8. Vorrichtung nach Anspruch 1, wobei die Vorrichtung zwei oder mehrere Vorratsbehälter (176, 182, 186, 192, 196, 450, 460, 470) umfasst.

9. Verfahren zum Behandeln von nassen Feststoffen, die von einem Fermentationsprozess mit einer oder mehreren Flüssigkeiten erhalten wurden, zum Bilden eines behandelten Feststoffs, wobei das Verfahren die folgenden Schritte beinhaltet:
(a) Bereitstellen einer Vorrichtung nach Anspruch 1;
(b) Aufbringen von nassen Feststoffen auf die erste Hauptfläche des Bandes, um eine Schicht von nassen Feststoffen zu bilden;
(c) Bewegen der Behandlungskammer von (i) einer ersten Position, in der die Behandlungskammer von dem Band getrennt ist, zu (ii) einer zweiten Position, in der die Behandlungskammer in Dichtungskontakt mit der ersten Hauptfläche des Bandes oder mit der Schicht von nassen Feststoffen ist;
(d) Aufbringen von einer oder mehreren Flüssigkeiten auf die Schicht von nassen Feststoffen, die unter der Behandlungskammer positioniert ist, während die Behandlungskammer in Dichtungskontakt ist; und
(e) wenigstens teilweises Entfernen der ein oder mehreren Flüssigkeiten von den nassen Feststoffen zum Bilden eines Abflusses.

10. Verfahren nach Anspruch 9, wobei Schritt (d) das sequentielle Aufbringen von zwei oder mehreren Flüssigkeiten auf die nassen Feststoffe beinhaltet.

11. Verfahren nach Anspruch 9, wobei Schritt (e) das Beaufschlagen der zweiten Hauptfläche des Bandes mit Unterdruck beinhaltet.

12. Verfahren nach Anspruch 9, wobei Schritt (d) unter Druck in der Behandlungskammer durchgeführt wird und/oder wobei Schritt (e) ohne Beaufschlagung von Unterdruck durchgeführt wird und/oder wobei, nach Schritt (e), das Verfahren ferner den Schritt des Bewegens der Behandlungskammer von der zweiten Position zur ersten Position und das Vorwärtsbewegen des Bandes um eine Distanz in Bahnabwärtsrichtung beinhaltet.

13. Verfahren zum Behandeln von nassen Feststoffen von einem Fermentationsprozess mit einer oder mehreren Flüssigkeiten zum Bilden eines behandelten Feststoffs, wobei das Verfahren die folgenden Schritte beinhaltet:
(a) Bereitstellen einer Vorrichtung nach Anspruch 5; wobei die Vorrichtung eine erste Behandlungskammer und eine zweite Behandlungskammer umfasst;
(b) Aufbringen von nassen Feststoffen auf die erste Hauptfläche des Bandes zum Bilden einer Schicht von nassen Feststoffen;
(c) Bewegen der ersten Behandlungskammer und der zweiten Behandlungskammer in Dichtungskontakt mit der ersten Hauptfläche des Bandes oder mit der Schicht von nassen Feststoffen;
(d) Aufbringen einer ersten Flüssigkeit auf die Schicht von nassen Feststoffen unter der ersten Behandlungskammer;
(e) sequentielles Aufbringen von zwei oder mehreren Flüssigkeiten auf die Schicht von nassen Feststoffen unter der zweiten Behandlungskammer;
(f) wenigstens teilweises Entfernen von wenigstens einem Teil der in den Schritten (d) und (e) aufgebrachten Flüssigkeiten von den nassen Feststoffen durch Beaufschlagen der zweiten Hauptfläche des Bandes mit Unterdruck;
(g) Bewegen der ersten und zweiten Behandlungskammer aus dem Dichtungskontakt mit dem Band; und
(h) Vorwärtsbewegen des Bandes um eine Distanz in der Bahnabwärtsrichtung.

14. Verfahren nach Anspruch 13, wobei die Schritte (b) bis (h) wiederholt werden, um einen kontinuierlichen Behandlungsprozess zu erzielen.

15. Verfahren nach Anspruch 13, wobei die Schritte (d), (e) und (f) während etwa derselben Zeitperiode durchgeführt werden, so dass nach Abschluss von Schritt (f) die nassen Feststoffe unter der ersten Behandlungskammer durch die erste Flüssigkeit behandelt wurden und die nassen Feststoffe unter der zweiten Behandlungskammer durch die zwei oder mehreren Flüssigkeiten behandelt wurden.

16. Verfahren nach Anspruch 9 oder Anspruch 13, wobei wenigstens eine der Flüssigkeiten ein Gemisch aus Ethanol und Wasser umfasst.

17. Verfahren nach Anspruch 9 oder Anspruch 13, wobei wenigstens eine der Flüssigkeiten einen Zusatz zum Behandeln der nassen Feststoffe beinhaltet, wie z.B. ein Vitamin, eine Farbe, ein Aroma, ein Verdaulichkeitshilfsmittel oder ein stromabwärtiges Verarbeitungshilfsmittel.

18. Verfahren nach Anspruch 9 oder Anspruch 13, wobei die Behandlung Wasser mit Ethanol verdrängt und/oder wobei die Behandlung ohne Erhöhen der Temperatur der nassen Feststoffe über eine Temperatur von etwa 150°C wie z.B. etwa 65°C durchgeführt wird.

## Revendications

1. Appareil de traitement d'une couche de solides mouillés obtenus par un processus de fermentation (330, 630) avec un ou plusieurs liquides, l'appareil comprenant :
(a) une courroie (162, 310) présentant une première surface principale (302) et une seconde surface principale (303) et une pluralité de pores s'étendant entre la première surface principale (302) et la seconde surface principale (303) pour conférer à la courroie (162, 310) une porosité aux un ou plusieurs liquides ;
(b) un système d'entraînement pour faire avancer la courroie (162, 310) par mouvements d'indexage dans un sens de production ;
(c) une chambre de traitement (320, 620) configurée pour se déplacer entre :
(i) une première position où la chambre de traitement (320, 620) est séparée de la courroie (162, 310) afin de permettre à la courroie (162, 310) d'être avancée par le système d'entraînement par mouvements d'indexage dans un sens de production ; et
(ii) une seconde position où la chambre de traitement (320, 620) est en contact hermétique avec la première surface principale (302) de la courroie (162, 310) ou avec la couche de solides particulaires positionnée sur la première surface principale (302) de la courroie (162, 310) ;
(d) une ou plusieurs buses dans la chambre de traitement (320, 620) pour appliquer un ou plusieurs liquides sur les solides mouillés (330, 630) ; et
(e) un ou plusieurs réservoirs (176, 182, 186, 192, 196, 450, 460, 470) pour renfermer un ou plusieurs liquides destinés au traitement des solides mouillés (330, 630) ; dans lequel les un ou plusieurs réservoirs (176, 182, 186, 192, 196, 450, 460, 470) sont en communication fluidique avec les une ou plusieurs buses.

2. Appareil selon la revendication 1, comportant en outre un système à vide (648) positionné près de la seconde surface principale (303) de la courroie (162, 310) pour éliminer au moins une partie du liquide appliqué sur les solides mouillés (330, 630) à travers les pores dans la courroie (162, 310) afin de former un effluent.

3. Appareil selon la revendication 1, dans lequel la courroie (162, 310) comporte des parties surélevées (380) agencées selon un motif sur la première surface principale (302) de la courroie (162, 310) de telle sorte qu'elles s'engagent avec la chambre de traitement (320, 620) pour former un joint entre la courroie (162, 310) et la chambre de traitement (320, 620).

4. Appareil selon la revendication 3, dans lequel les parties surélevées (380) sont moulées dans la courroie (162, 310).

5. Appareil selon la revendication 1, l'appareil comprenant deux ou plusieurs chambres de traitement (320, 620) agencées en série dans un sens de production par-dessus la courroie (162,310).

6. Appareil selon la revendication 1, l'appareil comprenant une chambre de traitement (320, 620) comprenant deux ou plusieurs sous-chambres (320a, 320b).

7. Appareil selon la revendication 1, dans lequel la chambre de traitement (320, 620) peut être pressurisée quand elle est en contact hermétique afin d'augmenter le flux de liquide à travers la couche de solides mouillés (330, 630) et la courroie (162, 310).

8. Appareil selon la revendication 1, l'appareil comprenant deux ou plusieurs réservoirs (176, 182, 186, 192, 196, 450, 460, 470).

9. Procédé de traitement de solides mouillés obtenus par un processus de fermentation avec un ou plusieurs liquides pour former un solide traité, le procédé comprenant les étapes consistant à :
(a) fournir un appareil selon la revendication 1 ;
(b) appliquer des solides mouillés sur la première surface principale de la courroie pour former une couche de solides mouillés ;
(c) déplacer la chambre de traitement de (i) une première position où la chambre de traitement est séparée de la courroie à (ii) une seconde position où la chambre de traitement est en contact hermétique avec la première surface principale de la courroie ou avec la couche de solides mouillés ;
(d) appliquer un ou plusieurs liquides sur la couche de solides mouillés positionnée sous la chambre de traitement pendant que la chambre de traitement est en contact hermétique ; et
(e) éliminer au moins partiellement les un ou plusieurs liquides des solides mouillés pour former un effluent.

10. Procédé selon la revendication 9, dans lequel l'étape (d) comprend l'application séquentielle de deux ou plusieurs liquides sur les solides mouillés.

11. Procédé selon la revendication 9, dans lequel l'étape (e) comprend l'application d'un vide sur la seconde surface principale de la courroie.

12. Procédé selon la revendication 9, dans lequel l'étape (d) est effectuée sous pression dans la chambre de traitement et/ou dans lequel l'étape (e) est effectuée sans l'utilisation du vide et/ou le procédé comportant en outre, après l'étape (e), l'étape de déplacement de la chambre de traitement de la seconde position à la première position et l'avancée de la courroie par une certaine distance dans le sens de production.

13. Procédé de traitement de solides mouillés obtenus par un processus de fermentation avec un ou plusieurs liquides pour former un solide traité, le procédé comprenant les étapes consistant à :
(a) fournir un appareil selon la revendication 5 ; l'appareil comprenant une première chambre de traitement et une seconde chambre de traitement ;
(b) appliquer des solides mouillés sur la première surface principale de la courroie pour former une couche de solides mouillés ;
(c) déplacer la première chambre de traitement et la seconde chambre de traitement en un contact hermétique avec la première surface principale de la courroie ou avec la couche de solides mouillés ;
(d) appliquer un premier liquide sur la couche de solides mouillés sous la première chambre de traitement ;
(e) appliquer séquentiellement deux ou plusieurs liquides sur la couche de solides mouillés sous la seconde chambre de traitement ;
(f) éliminer au moins partiellement au moins une partie des liquides appliqués aux étapes (d) et (e) des solides mouillés en appliquant un vide sur la seconde surface principale de la courroie ;
(g) déplacer les première et seconde chambres de traitement pour rompre leur contact hermétique avec la courroie ; et
(h) faire avancer la courroie par une certaine distance dans le sens de production.

14. Procédé selon la revendication 13, dans lequel les étapes (b) à (h) sont répétées pour assurer un processus de traitement continu.

15. Procédé selon la revendication 13, dans lequel les étapes (d), (e), et (f) sont effectuées durant approximativement la même période de temps de telle sorte qu'au terme de l'étape (f) les solides mouillés sous la première chambre de traitement aient été traités par le premier liquide, et les solides mouillés sous la seconde chambre de traitement aient été traités par les deux ou plusieurs liquides.

16. Procédé selon la revendication 9 ou la revendication 13, dans lequel au moins l'un des liquides comprend un mélange d'éthanol et d'eau.

17. Procédé selon la revendication 9 ou la revendication 13, dans lequel au moins l'un des liquides comprend un additif de traitement des solides mouillés, tel qu'une vitamine, un colorant, un arôme, un aide à la digestibilité ou un aide au traitement aval.

18. Procédé selon la revendication 9 ou la revendication 13, dans lequel le traitement déplace de l'eau avec de l'éthanol et/ou dans lequel le traitement est effectué sans augmenter la température des solides mouillés au-delà d'une température d'environ 150°C, telle qu'environ 65°C.
